# FASCICULE DE BREVET EUROPEEN

(11) **EP 3 921 321 B1**
(45) Date de publication et mention de la délivrance du brevet: **20.11.2024**
(21) Numéro de dépôt: 20702828.3
(22) Date de dépôt: 07.02.2020
(51) Int. Cl.: C07F 1/00, C07F 5/00, C07F 9/00, A61K 45/06, A61K 31/395, A61P 35/00

(54) **NOUVEAUX DÉRIVÉS AZOBENZÈNES, LEUR PROCÉDÉ DE PRÉPARATION ET LEUR UTILISATION POUR LE TRAITEMENT THÉRAPEUTIQUE ASSOCIÉ À DES RADIATIONS IONISANTES**
NEUE AZOBENZOLDERIVATE, VERFAHREN ZU IHRER HERSTELLUNG UND IHRE VERWENDUNG ZUR THERAPEUTISCHEN BEHANDLUNG VON IONISIERENDEN STRAHLEN
NOVEL AZOBENZENE DERIVATIVES, PROCESS FOR THEIR PREPARATION AND THEIR USE FOR THERAPEUTIC TREATMENT ASSOCIATED WITH IONIZING RADIATIONS

(30) Priorité: 08.02.2019 FR 1901285
(43) Date de publication de la demande: 15.12.2021
(73) Titulaire: Centre national de la recherche scientifique, 75016 Paris (FR); Université Paris-Saclay, 91190 Gif-sur-Yvette (FR); Institut Curie, 75248 Paris Cedex 05 (FR)
(72) Inventeur: BORT, Guillaume, 75007 PARIS (FR); COUVREUR, Patrick, 75016 PARIS (FR); MURA, Simona, 75012 PARIS (FR); POUZOULET, Frédéric, 91650 BREUILLET (FR)
(74) Mandataire: Novagraaf Technologies
(86) Numéro de dépôt international: PCT/EP2020/053144
(87) Numéro de publication internationale: WO 2020/161308

(56) Documents cités:
- CN-A- 104 447 598

## Description

La présente invention concerne le traitement thérapeutique de pathologies telles que le cancer, au moyen de composés activables par irradiations ionisantes.

Les systèmes redox et photosensibles ont été développés depuis plus d'un siècle et sont utilisés pour déclencher des actions complexes, telles que le clivage de liaison spécifique ou la commutation de configuration, qui peuvent être notamment utilisées pour effectuer la libération de molécules actives, contrôler l'activité de protéines et l'expression de gènes, etc... Malheureusement, les applications cliniques de ces systèmes sensibles sont encore limitées au traitement topique ou ophtalmique, en raison de la pénurie actuelle de techniques capables d'activer ces systèmes à une profondeur supérieure à quelques centaines de micromètres sous la surface corporelle, à l'exception de l'utilisation d'électrodes invasives ou de fibres optiques. En effet, toutes les molécules photosensibles développées jusqu'à présent sont sensibles uniquement aux irradiations UV, visibles ou proches infra-rouge, dont la pénétration dans les tissus est intrinsèquement limitée.

L'activation de composés déclenchée par des radiations ionisantes a été proposée notamment dans WO 2011/158189. Néanmoins, l'efficacité est extrêmement limitée, et nécessite des doses trop élevées pour être appliquée en clinique.

CN104447598A décrit des dérivés de polyamine macrocyclique de CA-4 ainsi qu'un sel pharmaceutiquement acceptable ou des isomères configurationnels des dérivés de polyamine macrocyclique. Les composés peuvent être utilisés pour inhiber la genèse ou la croissance d'une tumeur. Et, une composition médicamenteuse et l'application des composés dans le traitement de maladies tumorales.

Il est donc désirable de mettre à disposition de nouveaux composés qui peuvent être activés par des irradiations sans restriction de profondeur dans les tissus biologiques visés. Les irradiations par rayonnements ionisants peuvent être utilisées afin d'atteindre efficacement les tissus profonds, où la haute énergie des rayonnements sera convertie localement pour activer spécifiquement des systèmes thérapeutiques redox/photosensibles.

Selon l'invention, un convertisseur d'énergie moléculaire (chélate métallique) est associé à un commutateur photosensible (un groupement azobenzène) pour conduire à un système photosensible qui peut être activé par de la lumière à forte pénétration, à doses modérées compatibles avec les applications cliniques.

Selon un premier objet, la présente invention concerne donc un composé de formule (I) :

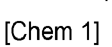

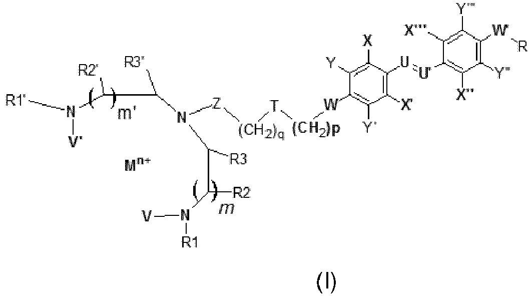

Dans laquelle :
M représente un atome de métal choisi parmi Ce(III), Pr(III), Nd(III), Sm(III), Eu(III), Gd(III), Tb(III), Dy(III), Ho(III), Er(III), Tm(III), Yb(III), Mg(ll), Ca(II), Mn(II), Fe(II), Fe(III), Cu(II), Zn(II), Ga(III), Y(III), Zr(III), Tc(IV), Tc (VI), Tc (VII), Ru(II), Ru(III), Ru(IV), Pd(II), Ag(I), In(III), Hf (IV), Re(VI), W(II), W(III), W(IV), W(V), W(VI), Os(III), Os(IV), Ir(III), Ir (IV), Pt(II), Au(I), Au (III), TI(III), Zr(IV), Nb(III), Bi(III) ;
n est égal à 1, 2, 3, 4, 5, 6 ou 7;
V et V' identiques ou différents sont des atomes d'hydrogène ou des chaînes alkoxy ou alkyle en C1-C10 linéaires ou ramifiées, ou des chaines alkyle en C1-C10 liées ensemble et comprenant un ou plusieurs hétéroatomes choisis parmi N, O ou S, optionnellement substituées par un ou plusieurs substituants choisis indépendamment parmi les atomes d'halogène, et les groupes nitrile, nitro, thio, amino, amido, aryle, hétéroaryle, hydroxyle, acide carboxylique ou carboxylate, pour former un cycle ;
R1, R1', R2, R2' identiques ou différents sont des atomes d'hydrogène, ou des chaines alkoxy ou alkyle en C1-C10 linéaires ou ramifiées éventuellement substituées par un ou plusieurs substituants choisis indépendamment parmi les atomes d'halogène, et les groupes nitrile, nitro, thio, amino, amido, aryle, hétéroaryle, hydroxyle, acide carboxylique ou carboxylate;
R3 et R3' identiques ou différents sont des atomes d'hydrogène ou des chaines alkoxy ou alkyle en C1-C10 linéaires ou ramifiées éventuellement substituées par un ou plusieurs substituants choisis indépendamment parmi les atomes d'halogène, et les groupes nitrile, nitro, thio, amino, amido, aryle, hétéroaryle, hydroxyle, acide carboxylique ou carboxylate, ou R3 et R3' sont liés ensemble pour former un hétérocycle ou hétéroaryle de 5 à 14 chaînons ;
m et m' identiques ou différents sont égaux à 1 ou 2 ;
X, X', X", X‴, Y, Y', Y", Y‴ identiques ou différents sont choisis indépendamment parmi H ; les atomes d'halogène ; les groupes alkoxy, alkyle ou cycloalkyle éventuellement interrompus ou substitués par un ou plusieurs hétéroatome(s) ou groupe COOH, CONH₂ , COSH, OH, NH₂, SH ; les groupes aryle ou hétéroaryle de 5 à 12 chaînons éventuellement substitués par un ou plusieurs groupe COOH, CONH₂, COSH ; les groupes COOH ou NH₂ ;
Z représente un groupe alkyle éventuellement interrompu ou substitué par un ou plusieurs hétéroatome(s) ou groupe COOH, CONH₂ , COSH, OH, NH₂, SH ; un groupes aryle ou hétéroaryle de 5 à 12 chaînons éventuellement substitué par un ou plusieurs groupe COOH, CONH₂, COSH ; ou un groupe COOH ou NH₂ ;
W et W' identiques ou différents représentent indépendamment un groupe CH₂; ou un groupe aryle ou cylcoalkyle ; un atome d'oxygène ou d'azote (secondaire ou ternaire) ; une liaison amide ; une liaison ester ; une liaison thioéther ;
U et U' identiques ou différents représentent le groupe CH ou NH, étant entendu que la double liaison U=U' est sous forme *cis* ou *trans ;*
T représente un groupe CH₂; un groupe -C(=O)NH ; un groupe alkoxy, alkyle ou cycloalkyl éventuellement interrompu ou substitué par un ou plusieurs hétéroatome(s) ou groupe COOH, CONH₂, COSH, OH, NH₂, SH; un groupe aryle ou hétéroaryle de 5 à 12 chainons contenant un ou plusieurs hétéroatome(s) et/ou éventuellement substitué par un ou plusieurs groupes choisis parmi COOalkyle, CONHalkyle, COSHalkyle ;
R représente H, ou chaine alkoxy ou alkyle en C1-C18 linéaire ou ramifiée, éventuellement substituée par un ou plusieurs substituants choisis indépendamment parmi les atomes d'halogène, et les groupes nitrile, nitro, thio, amino, amido, aryle, hétéroaryle, hydroxyle, acide carboxylique ou carboxylate ;
p et q sont des entiers compris entre 0 et 6, notamment entre 1 et 6 ;
Etant entendu que pour assurer la neutralité de la molécule, les n charges cationiques de M peuvent être neutralisées par de 0 à n groupes carboxylate (-COOH) éventuellement substitués sur R1, R1', R2, R2', R3, R3', V, V', et/ou de 0 à n des contre-ions présents en solution le cas échéant ;
Sous forme d'isomère *cis* et/ou *trans* et les mélanges de ceux-ci
Ou un sel pharmaceutiquement acceptable de celui-ci.

Les composés selon l'invention permettent d'induire la perméabilité et la mort des cellules cancéreuses lorsqu'ils sont activés par radiation ionisante. Après introduction de la forme inactive (*cis*), une irradiation du site cible par des radiations ionisantes est appliquée. Cette radiation induit une conversion de la molécule sous sa forme active *(trans)* conduisant à la perméabilité cellulaire et à la mort en raison de la modification de la balance lipophile de la molécule. Les rayons utilisés pour activer la molécule pénètrent au sein des tissus biologiques sans restriction de profondeur, d'où une technique d'activation parfaitement adaptée pour des applications cliniques, contrairement aux outils photosensibles traditionnels dont l'activation est limitée à des irradiations de l'UV à l'infrarouge proche (pénétration inférieure à 1 cm des tissus biologiques).

Les composés selon les inventions sont radiosensibles en ce que leur action est déclenchée par une radiation ionisante. Ils représentent donc un outil prometteur pour le contrôle et la modulation des actions thérapeutiques en temps réel.

A titre de métal M, on peut notamment citer les atomes métalliques dont les tailles sont comprises entre Z = 20 et Z = 83, et notamment les métaux suivants Fe, Cu, Zn, Ga, Y, Zr, Tc, Ru, Pd, Ag, In, Eu, Gd, Tb, Dy, Ho, Yb, Hf, W, Os, Ir, Pt, Au, Bi.

Dans la formule générale (I) précitée, les modes de réalisation suivants sont envisagés, pris séparément ou chacune de leur combinaison :
- V et V' sont liées ensemble au moyen d'une chaine alkyle en C1-C9, comprenant 2 atomes d'azote, éventuellement substituée par 2 groupes carboxylate ; et/ou
- V et V' sont liés de façon à former la chaîne : 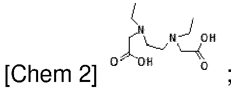 et/ou
- Z représente un groupe -CH(COOH)- ; et/ou
- m=m'=1 ; et/ou
- R2=R3=R2'=R3'=H ; et/ou
- R1=R1'=(CH2)COOH; et/ou
- p=q=2 ; et/ou
- Y=Y'=Y"=Y‴=H;
- W=W'=O ;
- U=U'=NH.

Selon un mode de réalisation, les composés de formule (I) obéissent à la formule générale (IA) :

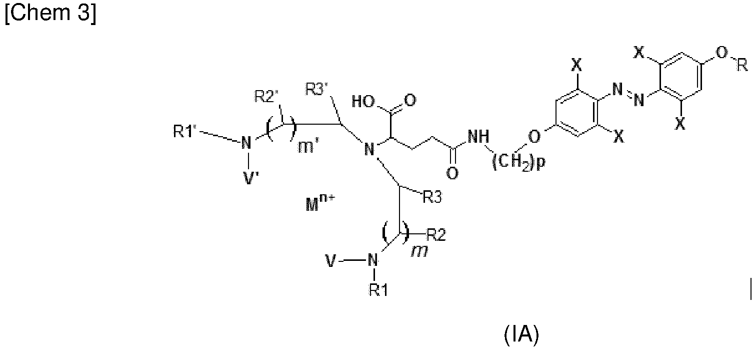

Dans laquelle :
M, n, R1, R2, R3, V, m, R1', R2', R3', V', m', p sont définis comme en formule (I) et
X est choisi parmi les atomes d'hydrogène et d'halogène ;
R est un groupe C1-C12 alkyle linéaire ou ramifié ;
La double liaison N=N est sous forme *cis* ou *trans ;*
Etant entendu que pour assurer la neutralité de la molécule, les n charges cationiques de M peuvent être neutralisées par de 0 à n groupes carboxylate (-COOH) substitués sur R1, R1', R2, R2', R3, R3', V, V', et/ou de 0 à n des contre-ions présents en solution le cas échéant,
Ou un sel pharmaceutiquement acceptable.

Selon un mode de réalisation, les composés de formule (I) obéissent à la formule générale (IB) : 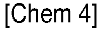 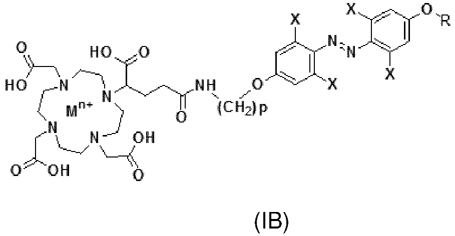 dans laquelle
M, n, p, X et R sont définis comme ci-avant, ou un sel pharmaceutiquement acceptable.

Selon un mode de réalisation, les composés de formule (I) obéissent à la formule générale (IC) : 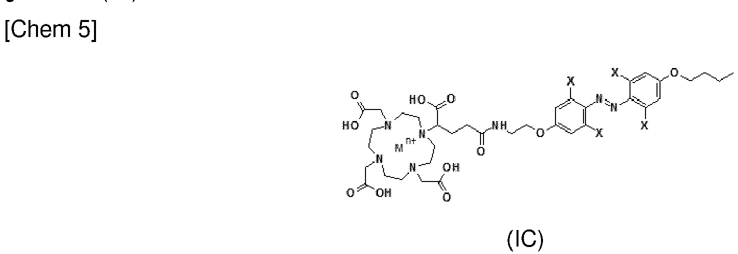 dans laquelle
M, n, X sont définis comme ci-avant, ou un sel pharmaceutiquement acceptable.

Selon un mode de réalisation, dans la formule (I), (IA) ou (IB) :
- X est choisi parmi H et F ; et/ou
- R représente un groupe C4-C8 alkyle linéaire ;
- p=2;
- M est un métal choisi parmi Cu, Ga, Y, In, Eu, Gd, Yb, Bi ;
- n=2 ou 3.

Dans ce qui précède et ce qui suit :
« Alkyle » désigne un groupe hydrocarboné aliphatique qui peut être linéaire ou ramifié ayant environ 1 à environ 20 atomes de carbone dans la chaîne. Des groupes alkyle préférés ont 1 à environ 12 atomes de carbone dans la chaîne. Ramifié signifie qu'un ou plusieurs groupes alkyle inférieurs, tels que le méthyle, l'éthyle ou le propyle, sont liés à une chaîne alkyle linéaire.

« Alkoxy » désigne un groupe alkyl-O-, dans lequel le groupe alkyle est tel que décrit ci-avant. Des exemples types de groupes alkoxy comprennent le méthoxy, l'éthoxy, le *n-*propoxy, l'*i*-propoxy, le *n*-butoxy et l'heptoxy.

Cycloalkyle" signifie un système de cycle non aromatique mono- ou multicyclique d'environ 3 à environ 10 atomes de carbone, de préférence d'environ 5 à environ 10 atomes de carbone. Les tailles de cycle préférées des cycles du système de cycle comprennent environ 5 à environ 6 atomes de cycle. Les cycloalkyles monocycliques exemplaires comprennent le cyclopentyle, le cyclohexyle, le cycloheptyle.

« Carboxy » désigne un groupe HO(O)C- (acide carboxylique).

« Aryle » désigne un système cyclique monocyclique ou multicyclique aromatique à environ 6 à environ 14 atomes de carbone, de préférence à environ 6 à environ 10 atomes de carbone. Des exemples types de groupes aryle comprennent le phényle ou le naphtyle.

"Hétéroaryle" signifie un système de cycle monocyclique ou multicyclique aromatique d'environ 5 à environ 14 atomes de carbone, de préférence d'environ 5 à environ 10 atomes de carbone, dans lequel un ou plusieurs des atomes de carbone dans le système de cycle est/sont un(des) hétéro élément(s) différents du carbone, par exemple l'azote, l'oxygène ou le soufre. Les tailles de cycle préférées des cycles du système de cycle comprennent environ 5 à environ 6 atomes de cycle. Les groupes hétéroaryle exemplaires comprennent le pyrazinyle, le thiényle, l'isothiazolyle, l'oxazolyle, le pyrazolyle, le furazanyle, le pyrrolyle, le 1,2,4-thiadiazolyle, le pyridazinyle, le quinoxalinyle, le phtalazinyle, l'imidazo[1,2-a]pyridine, l'imidazo[2,1-b]thiazolyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzthiazolyle, le furanyle, l'imidazolyle, l'indolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le pyrrolyle, le quinazolinyle, le quinolinyle, le 1,3,4-thiadiazolyle, le thiazolyle, le thiényle et le triazolyle.

« Halogène » signifie fluore, chlore, brome ou iode. Sont préférés le fluore, le chlore ou le brome, et plus particulièrement le fluore.

La présente invention concerne également l'utilisation thérapeutique des composés selon l'invention.

Selon un autre objet, la présente invention concerne les compositions pharmaceutiques comprenant un composé de formule (I) majoritairement sous forme *cis,* et au moins un excipient pharmaceutiquement acceptable.

Selon un autre objet, la présente invention concerne un composé de formule (I) sous forme *cis* et/ou *trans* pour son utilisation pour le traitement du cancer.

Les composés de formule (I) ci-dessous sont activables par une irradiation ionisante, la double liaison *cis* commutant sous la forme *trans,* cytotoxique.

Ladite utilisation comprend donc l'administration d'un composé de formule (I) sous forme majoritaire *cis,* et l'application d'irradiations au site cible, de façon à former *in situ* le composé de forme *trans.*

Ladite utilisation comprend également le suivi du traitement par imagerie *in vivo* par IRM, TEP, scanner X ou TEMP.

Selon un mode de réalisation, ladite utilisation comprend l'administration d'une quantité efficace d'un composé de formule (I), sous forme *cis,* à un patient qui en a besoin.

Selon un mode de réalisation, l'administration dudit composé de formule (I) peut généralement s'effectuer par voie parentérale.

Les radiations ionisantes envisagées correspondent généralement aux radiations utilisées pour les traitements par radiothérapie. De telles actions induites par un stimulus externe permettent le contrôle temporel et spatial et peut être ajusté à la demande en temps réel afin d'obtenir une efficacité thérapeutique optimale. On peut notamment citer les rayonnements ionisants tels que les photons (rayons X et γ dont l'énergie peut aller de 1 keV à 25 MeV), les électrons et hadrons (tels que les protons ou les carbones de 60 à 300 MeV), et notamment les rayons X (1-200 keV), les rayons gamma (662 keV) et les faisceaux d'électrons (4500 keV).

Ces rayonnements permettent d'obtenir l'activation souhaitée à des doses faibles comprises entre 2 et 20 Gy, notamment comprises entre 2 et 10 Gy, plus particulièrement entre 2 et 5 Gy.

L'action des composés de l'invention s'exerçant par la perméabilité cellulaire, tous les cancers peuvent être potentiellement traités par les composés de l'invention, dans la mesure où l'approche thérapeutique n'est pas limitée à une signature de la surface cellulaire spécifique. On peut notamment citer le cancer du poumon, le cancer du pancréas, le cancer du foie, le cancer de la rate, le carcinome pulmonaire à petites cellules, le cancer de la prostate, le rhabdomyosarcome, le cancer de l'estomac, le cancer gastrointestinal, le cancer colorectal, le cancer du rein, le cancer du sein, le cancer des ovaires, le cancer du testicule, le cancer de la thyroïde, le cancer de la tête et du cou, le cancer de la peau, le sarcome des tissus mous, le carcinome de la vessie, les cancers osseux, le myélome, le plasmocytome, le cancer des cellules germinales, le cancer de l'utérus, les leucémies, les lymphomes, le neuroblastome, le ostéosarcome, le rétinoblastome, les cancers du système nerveux central, les tumeurs de Wilms et particulièrement le cancer du pancréas ou la leucémie.

Selon un mode de réalisation, l'utilisation permet également de détecter la présence du composé et éventuellement sa commutation moléculaire *in vivo* par IRM grâce à la présence d'atome(s) métallique(s) dans la structure moléculaire des composés de l'invention.

La détection par IRM des composés de l'invention permet la localisation et la surveillance de l'action thérapeutique desdits composés de façon non-invasive, facilitant ainsi le développement et le suivi des protocoles thérapeutiques, et permettant ainsi une approche théranostique.

Selon un mode de réalisation, les composés de l'invention peuvent être administrés en combinaison avec un ou plusieurs agents anti-cancéreux, tels qu'une chimiothérapie ou une immunothérapie comme les inhibiteurs de checkpoints immunitaires tels que les anti-CTLA4, anti-PD-L1 et anti-PD1.

"Quantité efficace" signifie une quantité d'un composé/composition selon la présente invention efficace dans la production de l'effet thérapeutique souhaité.

"Formulations adaptées pour une administration parentérale" signifie des formulations qui sont sous une forme adaptée pour être administrées par voie parentérale à un patient. Les formulations sont stériles et comprennent les émulsions, les suspensions, les solutions d'injection aqueuses et non aqueuses, qui peuvent contenir des agents de suspension et des agents épaississants et des anti-oxydants (après avoir vérifié que l'effet potentiellement radioprotecteur de ceux-ci n'empêchent pas l'effet recherché sous rayonnement), des tampons, des bactériostatiques et des solutés qui rendent la formulation isotonique, et ont un pH ajusté de manière adaptée, avec le sang du receveur voulu.

La formulation est de préférence administrée par injection, y compris transmusculaire, intraveineuse, intrapéritonéale, et sous-cutanée. Pour l'injection, les composés utiles selon l'invention peuvent être formulés en solutions liquides, de préférence dans des tampons physiologiquement compatibles tels que la solution de Hank ou la solution de Ringer. De plus, les composés peuvent être formulés sous forme solide et redissout ou suspendus immédiatement avant l'utilisation. Les formes lyophilisées sont aussi comprises.

Le choix du véhicule et le contenu de la substance active dans le véhicule sont généralement déterminés selon la solubilité et les propriétés chimiques du composé actif, le mode d'administration particulier et les dispositions à observer dans la pratique pharmaceutique.

Les formulations peuvent être préparées sous forme pharmaceutique unitaire par l'un quelconque des procédés bien connus dans l'art de la pharmacie. Ces procédés comprennent l'étape consistant à associer l'ingrédient actif et le transporteur qui constitue un ou plusieurs ingrédients accessoires. En général, les formulations sont préparées en associant de façon uniforme et intime l'ingrédient actif et les transporteurs liquides ou les transporteurs solides finement fractionnés ou les deux, puis, si nécessaire, en façonnant le produit.

Les niveaux de dosages réels d'un ingrédient actif dans les compositions de l'invention peuvent varier afin d'obtenir une quantité d'ingrédient actif efficace pour obtenir une réponse thérapeutique souhaitée pour une composition particulière et un mode d'administration. Le niveau de dosage choisi par conséquent dépend de l'effet thérapeutique souhaité, du mode d'administration, de la durée du traitement souhaitée et d'autres facteurs.

Les compositions à unité posologique peuvent contenir de telles quantités de tels sous-multiples de ceux-ci tout comme elles peuvent être utilisées pour composer la dose quotidienne. Il sera compris toutefois que le niveau de dose spécifique pour tout patient particulier dépendra d'une variété de facteurs comprenant le poids corporel, la santé générale, le sexe, l'alimentation, le moment et le mode d'administration, les vitesses d'absorption et d'excrétion, l'association à d'autres médicaments et la gravité de la maladie traitée.

La quantité de chaque composant administré est déterminée par les médecins traitants en tenant compte de l'étiologie et de la gravité de la maladie, l'état et l'âge du malade, la puissance de chaque composant et autres facteurs.

Les formulations peuvent être présentées en conteneurs unidoses ou multidoses, par exemple ampoules et flacons scellés avec bouchons élastomères et peuvent être stockées sous forme lyophilisée nécessitant uniquement l'ajout d'un transporteur liquide stérile, par exemple de l'eau pour les injections, juste avant l'usage. Les solutions en injections et suspensions préparées extemporanément peuvent être préparées à partir de poudres stériles, granules et comprimés du type décrit précédemment.

« Forme de dose liquide" signifie que la dose du composé actif à administrer au patient est sous forme liquide, par exemple, des émulsions, des solutions, des suspensions, des sirops et des élixirs pharmaceutiquement acceptables. En plus des composés actifs, les formes de dose liquide peuvent contenir des diluants inertes couramment utilisés dans la technique, tels que l'eau ou d'autres solvants, des agents solubilisants et des émulsifiants, comme par exemple, l'alcool d'éthyle, l'alcool d'isopropyle, l'éthyl carbonate, l'éthyl acétate, l'alcool de benzyle, le benzoate de benzyle, le propylèneglycol, le 1,3-butylèneglycol, le diméthylformamide, les huiles (après avoir vérifié que l'effet potentiellement radioprotecteur de celles-ci n'empêchent pas l'effet recherché sous rayonnement), en particulier l'huile de coton, l'huile d'arachide, l'huile de germe de maïs, l'huile d'olive, l'huile de castor et l'huile de sésame, le glycérol, l'alcool de tétrahydrofurfuryle, les polyéthylèneglycols et les esters d'acide gras de sorbitan ou des mélanges de ces substances, et similaires.

"Patient" comprend à la fois un humain et d'autres mammifères.

"Composition pharmaceutique" signifie une composition comprenant un composé de formule I et au moins un composant choisi dans le groupe comprenant les transporteurs, diluants, adjuvants, excipients, ou véhicules pharmaceutiquement acceptables, tels que les agents de conservation, les charges, les agents mouillants, les agents émulsifiants, les agents de suspension, les agents antibactériens, les agents antifongiques, les agents lubrifiants, et les agents dispersants, selon la nature du mode d'administration et les formes de dosage. Les exemples d'agents de suspension comprennent les alcools isostéaryle éthoxylés, le polyoxyéthylène sorbitol et les esters de sorbitan, la cellulose microcristalline, le métahydroxyde d'aluminium, la bentonite, l'agar-agar et la tragacanthe, ou des mélanges de ces substances. La prévention de l'action des microorganismes peut être assurée par divers agents antibactériens et antifongiques, par exemple les parabènes, le chlorobutanol, le phénol, l'acide sorbique, et similaires. Il peut aussi être souhaitable de comprendre les agents isotoniques, par exemple les sucres, le chlorure de sodium et similaires. Les exemples de transporteurs, diluants, solvants ou véhicules adaptés comprennent l'eau, l'éthanol, les polyols, leurs mélanges adaptés, les huiles végétales (telles que l'huile d'olive) et les esters organiques injectables tels que l'éthyl oléate.

"Pharmaceutiquement acceptable" signifie que c'est, dans la portée d'un jugement médical valable, adapté pour une utilisation en contact avec les cellules des humains et des animaux inférieurs sans toxicité, irritation, réponse allergique indue et similaires, et sont proportionnés à un rapport avantage/risque raisonnable.

L'expression « sels pharmaceutiquement acceptables » fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels décrits par exemple S.M. Berge et al. « Pharmaceutical Salts » J. Pharm. Sci, 66 :p.1-19 (1977). Les sels d'addition acide peuvent également être préparés en faisant réagir séparément le composé purifié sous sa forme acide avec une base organique ou inorganique et en isolant le sel ainsi formé. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical.

On doit comprendre que la présente invention couvre toutes les combinaisons appropriées des groupements particuliers et préférés mentionnés ici.

Selon un autre objet, la présente invention concerne également le procédé de préparation des composés de formule (I), ledit procédé comprenant :
- l'étape de complexation du composé de formule (II) :

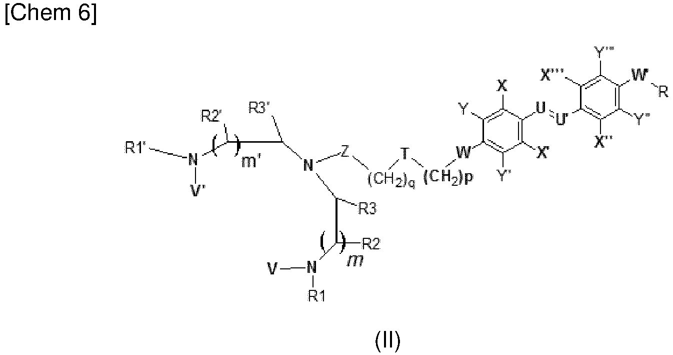

Dans laquelle :
R1, R2, R3, V, m, R1', R2', R3', V', m', Z, T, q, p, W, W', X, X', X", X‴, Y, Y', Y", Y‴, R, U, U' sont tels que définis ci-avant,
Sous forme d'isomère *cis* et/ou *trans* et les mélanges de ceux-ci ;
avec un précurseur du métal M, et
   - l'irradiation UV éventuelle du produit obtenu afin d'obtenir l'isomère *cis* majoritairement.

La complexation avec l'agent métallique M peut être généralement réalisée dans un solvant tel que l'eau, à température comprise entre la température ambiante et la température de reflux du mélange réactionnel, par exemple entre 40 et 100°C. Typiquement, le pH de la solution est maintenu entre 5 et 7.

Selon un mode de réalisation, le composé de formule (II) répond à la formule (IIA) :

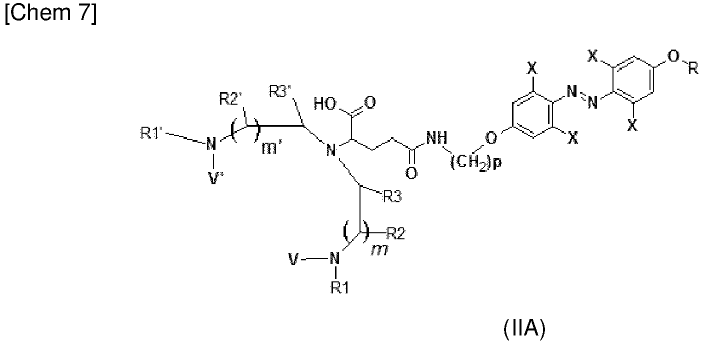

Dans laquelle R1, R2, R3, V, m, R1', R2', R3', V', m', p, X, R sont définis comme en formule (I).

Selon un mode de réalisation, le composé de formule (II) répond à la formule (IIB) :

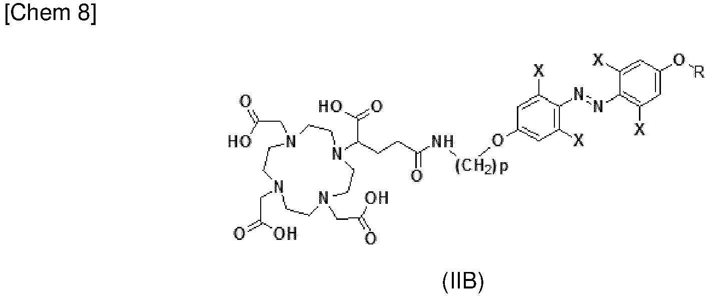

Dans laquelle p, X, R sont définis comme en formule (I).

Selon un mode de réalisation, le composé de formule (II) répond à la formule (IIC) :

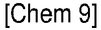

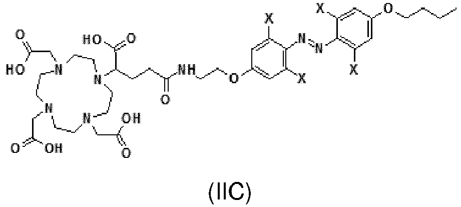

Dans laquelle X est défini comme en formule (I).

Selon un mode de réalisation, le composé de formule (II) est obtenu par couplage d'un composé de formule (III) et d'un composé de formule (IV) :

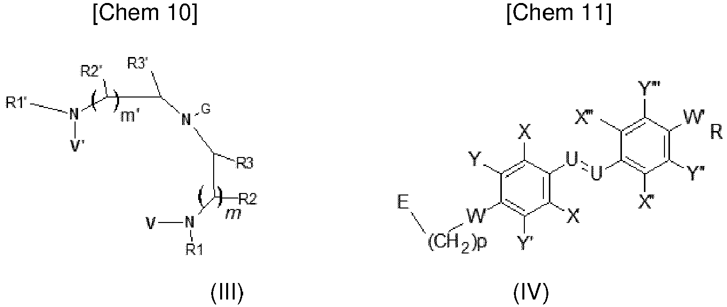

Dans lesquelles :
R1, R2, R3, V, m, R1', R2', R3', V', m', Z, T, q, p, W, W', X, X', X", X‴, Y, Y', Y", Y‴, R, U, U' sont tels que définis ci-avant; et
E est une chaîne alkoxy ou alkyle en C1-C10 linéaire ou ramifiée, un groupe cycloalkyle ou aryle, comprenant éventuellement un ou plusieurs hétéroatomes choisis parmi N, O ou S, éventuellement intégrant ou substituée par un ou plusieurs substituants choisis indépendamment parmi les atomes d'halogène, et les groupes anhydride, carboxy, nitrile, nitro, thio, amino, amido, aryle, hétéroaryle, hydroxyle, ester, acide carboxylique ou carboxylate ; étant entendu que E contient un substituant nucléophile tel qu'une fonction hydroxyle, thiol ou une amine primaire ou secondaire pour effectuer le couplage sur la molécule (III).

G est une chaîne alkoxy ou alkyle en C1-C10 linéaire ou ramifiée, un goupe cylcoalkyle ou aryle, comprenant éventuellement un ou plusieurs hétéroatomes choisis parmi N, O ou S, éventuellement substituées par un ou plusieurs substituants choisis indépendamment parmi les atomes d'halogène, et les groupes anhydride, carboxy, nitrile, nitro, thio, amino (primaire ou secondaire), amido, aryle, hétéroaryle, hydroxyle, acide carboxylique ou carboxylate, étant entendu que G contient un substituant électrophile tel qu'une fonction acide carboxylique activée ou une fonction anhydride pour effectuer le couplage sur la molécule (IV).

La réaction de couplage peut-être réalisée en présence d'une base, notamment une base organique telle que la triéthylamine par exemple. La réaction peut être conduite sous conditions anhydre.

Selon un mode de réalisation, le composé de formule (III) répond à la formule (IIIA) :

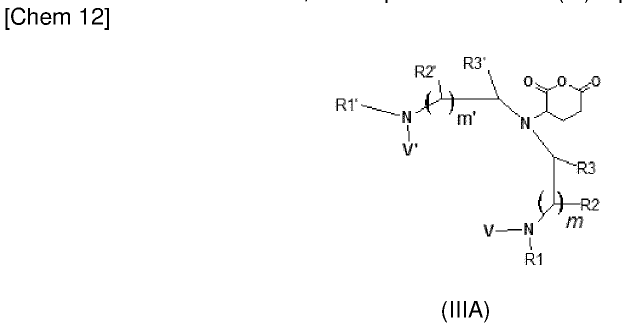

Dans laquelle R1, R2, R3, V, m, R1', R2', R3', V', m', sont tels que définis ci-avant.

Selon un mode de réalisation, le composé de formule (III) répond à la formule (IIIB) :

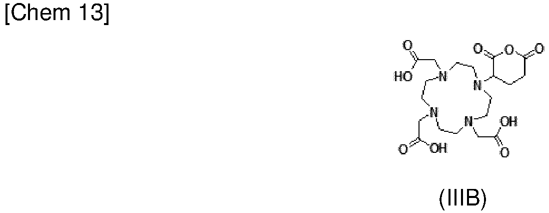

Selon un mode de réalisation, le composé de formule (IV) répond à la formule (IVA) :

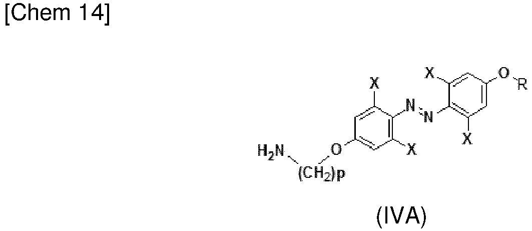

Dans laquelle p, X, R sont tels que définis en formule (I).

Selon un mode de réalisation, le composé de formule (IV) répond à la formule (IVB) :

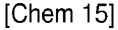

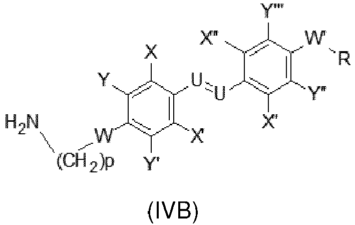

Dans laquelle p, W, W', X, X', X", X‴, Y, Y', Y", Y‴, R, U, U' sont tels que définis ci-avant. Selon un mode de réalisation, le composé de formule (IV) répond à la formule (IVC) :

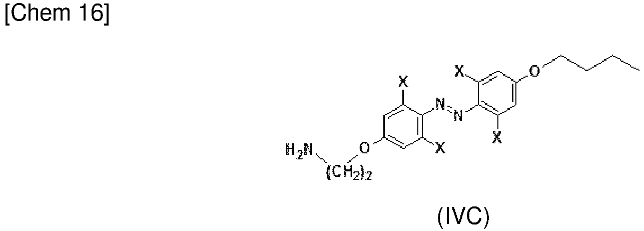

Dans laquelle X est tel que défini en formule (I).

Les composés de formule (III) sont généralement commerciaux. En particulier, le composé de formule (IIIB) est le composé DOTAGA.

Les composés de formule (IV) peuvent être préparés par application et/ou adaptation de la méthode de synthèse décrite ci-après dans les exemples.

De façon générale, les produits de départ et intermédiaires des composés utiles selon l'invention peuvent être préparés par l'application ou l'adaptation de procédés connus utilisés jusque-là ou décrits dans la littérature, par exemple ceux décrits par R.C. Larock dans Comprehensive Organic Transformations, VCH publishers, 1989.

Dans les réactions, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Green et P.G.M. Wuts dans « Protective Groups in Organic Chemistry » John Wiley and Sons, 1991 ; J.F.W. McOmie dans « Protective Groups in Organic Chemistry » Plenum Press, 1973.

Sauf indication spécifique, il n'y a pas de restriction particulière sur la nature du solvant à employer, à condition qu'il n'ait aucun effet indésirable sur la réaction ou sur les réactifs impliqués. Les exemples de solvants adaptés comprennent : hydrocarbures, qui peuvent être des hydrocarbures aromatiques, aliphatiques ou cycloaliphatiques, tels que hexane, cyclohexane, benzène, toluène et xylène ; amides, en particulier les amides d'acides gras tels que diméthylformamide et les éthers, tels que l'éther diéthylique et le tétrahydrofuranne.

Les réactions peuvent avoir lieu à une vaste gamme de températures, généralement à une température entre environ 0°C et 150°C (de préférence entre environ une température ambiante et environ 100°C). Le temps requis pour la réaction peut également varier considérablement, selon de nombreux facteurs, notamment la température de réaction et la nature des réactifs.

Les composés ainsi préparés peuvent être récupérés à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.]

Les sels d'additions acides peuvent être formés avec les composés utiles selon l'invention dans lesquels une fonction de base tels qu'un groupe amino, akylamino ou dialkylamino est présente. Les sels d'addition acide pharmaceutiquement acceptables, c'est-à-dire non toxiques, sont préférés. Les sels sélectionnés sont choisis de façon optimale pour être compatibles avec les véhicules pharmaceutiques habituels et adaptés pour l'administration parentérale. Les sels d'addition acide des composés utiles selon cette invention peuvent être préparés par réaction de la base libre avec l'acide approprié, par l'application ou l'adaptation de procédés connus. Par exemples, les sels d'addition acide des composés utiles selon cette invention peuvent être préparés soit en dissolvant la base libre dans de l'eau ou dans une solution aqueuse alcoolisée ou des solvants adaptés contenant l'acide approprié et en isolant le sel en évaporant la solution, ou en faisant réagir la base libre et l'acide dans un solvant organique, auquel cas le sel se sépare directement ou peut être obtenu par concentration de la solution. Les sels d'addition acide des composés utiles selon cette invention peuvent être régénérés à partir des sels par l'application ou l'adaptation de procédés connus. Par exemple, les composés parents utiles selon l'invention peuvent être régénérés à partir de leurs sels d'addition acide par traitement avec un alkali, par exemple une solution de bicarbonate de sodium aqueuse ou une solution d'ammoniac aqueuse.

Les composés utiles selon cette invention peuvent être régénérés à partir de leurs sels d'addition de base par l'application ou l'adaptation de procédés connus. Par exemple, les composés parents utiles selon l'invention peuvent être régénérés à partir de leurs sels d'addition de base par le traitement avec un acide, par exemple un acide chlorhydrique. Les sels d'addition de base peuvent être formés lorsque le composé utile selon l'invention contient un groupe carboxyle. Les bases qui peuvent être utilisées pour préparer les sels d'addition de base comprennent de préférence celles qui produisent, lorsqu'elles sont associées à un acide libre, des sels pharmaceutiquement acceptables, c'est-à-dire des sels dont les cations ne sont pas toxiques pour le patient dans les doses pharmaceutiques des sels, de sorte que les effets inhibiteurs bénéfiques inhérents à la base libre ne soient pas annulés par les effets secondaires imputables aux cations. Les sels pharmaceutiquement acceptables, comprenant ceux dérivés des sels de métal alcalino-terreux, dans la portée de l'invention comprennent ceux dérivés des bases suivantes : hydrure de sodium, hydroxyde de sodium, hydroxyde de potassium, hydroxyde de calcium, hydroxyde d'aluminium, hydroxyde de lithium, hydroxyde de magnésium, hydroxyde de zinc, ammoniac, éthylènediamine, N-méthyl-glucamine, lysine, arginine, ornithine, choline, N,N'-dibenzylethylenediamine, chloroprocaïne, diéthanolamine, procaïne, N-benzylphénéthylamine, diéthylamine, pipérazine, tris(hydroxymethyl)-aminomethane, hydroxyde de tétraméthylammonium et analogues. Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation d'un mélange de solvant aqueux/organique, en utilisant des solvants organiques tels que dioxan, tétrahydrofuranne ou méthanol.

### Figures :

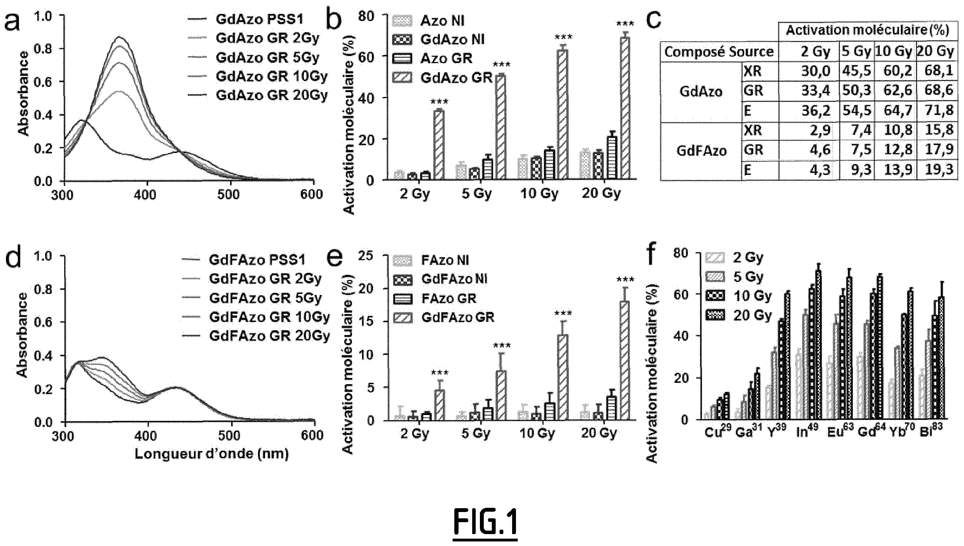

[Fig 1] La figure 1 représente les spectres d'absorbance des composés *cis***-GdAzo (a)** et *cis***-GdFAzo (d)** sous irradiation gamma dans le PBS. PPS1 : Etat photostationnaire contenant l'isomère *cis* majoritaire. **b, e** : Les histogrammes représentant l'activation moléculaire du *cis***-GdAzo** et du *cis*-**Azo** (molécule contrôle sans Gd) **(b)** ou du *cis-***GdFAzo** et du *cis*-**FAzo** (molécule contrôle sans Gd) **(e)** sous irradiation gamma dans le PBS déterminée par HPLC et calculée par la différence de proportion en isomère *trans* dans le milieu (n=3). **c** : Activation moléculaire de cis-GdAzo et *cis*-**GdFAzo** sous rayons-X, gamma et accélérateur linéaire d'électrons Linac. **f** : Histogramme représentant l'activation moléculaire de *cis*-**MAzo (M** étant le métal indiqué en abscisse) sous irradiation gamma dans le PBS déterminée par HPLC et calculée par la différence de proportion en isomère *trans* dans le milieu (n=3). Gy : Gray (J.L⁻¹). XR : Irradiation par rayons X. GR : Irradiation par rayons gamma. E : Irradiation par accélérateur linéaire d'électrons Linac. NI : non irradié. Les écarts-types standards (*standard déviations*) sont représentés sur les graphes. *** *P <* 0,001 (*Two-wayAnova with Bonferroni post*-*test*)*.*

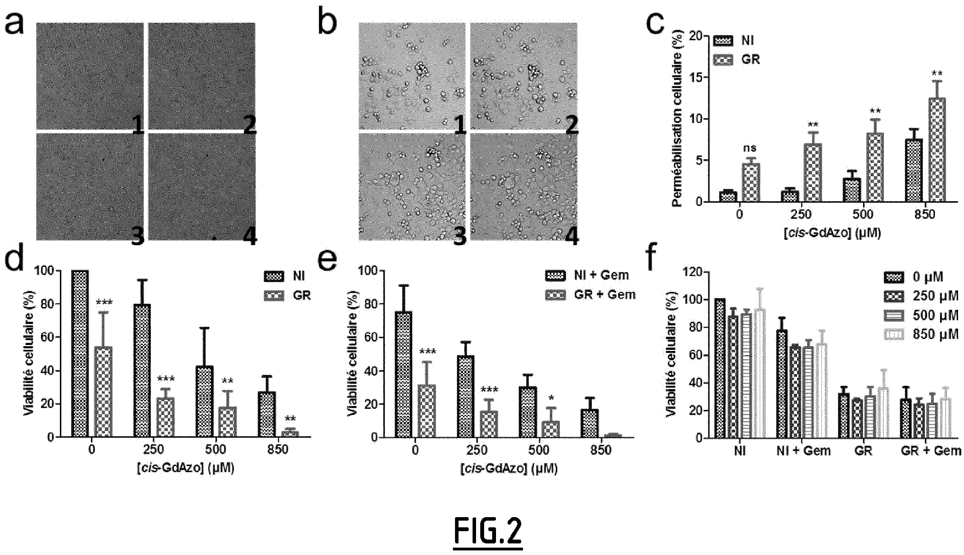

[Fig 2] La Figure 2 représente **a** : Images en microscopie de cellules cancéreuses (Panc-1, cancer pancréatique humain) en présence d'iodure de propidium et du composé inactif *cis*-**GdAzo** (1, 2) ou du composé actif *trans*-**GdAzo** (3, 4) à une concentration de 500 µM avant (1, 3) ou 30 min après (2, 4) l'introduction des composés. Superposition des clichés obtenus en lumière blanche et en fluorescence (l'iodure de propidium dans les noyaux apparait en rouge et montre la perméabilisation membranaire). **b** : Images en microscopie de cellules cancéreuses (Panc-1) en présence d'iodure de propidium et du composé inactif *cis-***GdAzo** à une concentration de 250 µM (1, 2) ou 500 µM (3, 4), avant (1, 3) ou 30 min après (2, 4) irradiation du milieu sous rayonnement gamma (2 Gy). **c** : Histogramme comparant l'impact de l'irradiation par rayonnements gamma (2 Gy) sur la perméabilisation cellulaire (Panc-1) en présence d'iodure de propidium et de *cis*-**GdAzo** (n=3). **d, e** : Histogrammes comparant l'impact de l'irradiation par rayonnements gamma (2 Gy) sur la mortalité de cellules cancéreuses résistantes à la Gem (CCRF-CEM ARAC-8C, leucémie lymphoblastique aiguë humaine) 4 jours après traitement en présence de *cis*-**GdAzo** (**d)** ou en présence de *cis*-**GdAzo** et de Gem (0,1 µM) **(e)** pendant une durée de 1 h. **f** : Histogramme comparant l'impact de l'irradiation par rayonnements gamma (2 Gy) sur la mortalité de cellules cancéreuses résistantes à la Gem (CCRF-CEM ARAC-8C) en présence de Dotarem^{®} ou de Dotarem^{®} et de Gem (0,1 µM). Gy : Gray (J.L⁻¹). GR : Irradiation par rayons gamma. NI : non irradié. Les erreurs standards sur la moyenne (*standard error of the mean*) sont représentées sur le graphe **c** et les écarts-types standards (*standard déviations*) sont représentés sur les graphes **d-f.** * *P* < 0,05, ** *P* < 0,01, *** *P* < 0,001 (*Two-way Anova with Bonferroni post-test*)*.*

### Exemples

### I. Méthodes de chromatographies analytiques.

### Méthode A

Les analyses HPLC (high-performance liquid chromatography) étaient effectuées sur une pompe HPLC binaire 1565 (Waters), lecteur PAD 2998 (Waters), avec une colonne phase inverse Agilent Eclipse XDB-C18 (longueur: 250 mm, diamètre: 4,6 mm, phase stationnaire: 5 µm) en utilisant un système de gradient eau-TFA 0,05%/acétonitrile-TFA 0,05%; 0' (98/2), 5' (98/2), 25' (0/100), 27' (0/100), 29' (98/2), 35' (98/2) à un débit de 1 mL.min⁻¹ (30 µL injecté). Les données étaient traitées sur Empower.

### Méthode B

Les analyses LCMS (liquid chromatography mass spectrometry) étaient effectuées sur un système Alliance 2695 (Waters) avec une colonne phase inverse XBridge C18 (longueur: 150 mm, diamètre: 2,1 mm, phase stationnaire: 3,5 µm) en utilisant un système de gradient eau-FA 0.1%/acétonitrile; 0' (95/5), 20' (0/100) à un débit de 0,25 mL.min⁻¹ (10 µL injecté). L'analyseur massique était un TOF LCT Premier (Waters). La tension du capillaire était de 2,8 kV. La tension de cône était de 35 V. La température de la source était de 120 °C et la température de désolvatation était de 280 °C. Les données étaient traitées sur MassLynx.

### Méthode C

Les analyses HPLC étaient effectuées sur une pompe HPLC binaire 2525 (Waters) couplée à une pompe HPLC 515 (Waters), lecteur PAD 2996 (Waters), avec une colonne phase inverse XBridge C18 (longueur: 100 mm, diamètre: 3,0 mm, phase stationnaire: 3,5 µm) en utilisant un système isocratique eau-TFA 0,05%/acétonitrile à un débit de 0,75 mL.min⁻¹ (10 µL injecté). La longueur d'onde de détection correspondait au point isobestique du composé étudié dans la phase mobile utilisée. Les données étaient traitées sur MassLynx.

### II. Synthèses.

### Général

Tous les réactifs ont été achetés chez Sigma-Aldrich ou Alfa Aesar avec la pureté maximale disponible et ont été utilisés sans purification complémentaire. L'anhydride de DOTAGA a été acheté chez CheMatech et a été utilisé sans purification complémentaire. Le gel de silice (Aldrich 717185 Si 60, 40-63 µm) utilisé pour les chromatographies flash a été acheté chez VWR. Les chromatographies flash en phase inverse RP-18 ont été effectuées sur un appareil CombiFlash (Biotage). Les chromatographies sur couches minces ont été effectuées par l'utilisation de feuilles d'aluminium recouvertes de gel de silice 60F254 (détection par lampe UV à 254 nm ou par ninhydrine). Les spectres RMN ¹H, ¹³C et ¹⁹F ont été acquis sur des spectromètres Bruker 300 MHz ou 400 MHz à ta. Les déplacements chimiques δ sont donnés en ppm en utilisant le solvant comme référence. Les constantes de couplage J sont mesurées en Hz. Les profils de couplage sont décrits par les abréviations d (doublet), t (triplet) et m (mutiplet). Les expériences de spectrométrie de masse en haute résolution (HRMS) ont été effectuée dans un mélange H₂O/méthanol 3/7 par ionisation électrospray en mode positif, sauf si indication contraire, par l'utilisation d'un analyseur de masse en temps de vol avec un spectromètre de masse TOF-LCT Premier (Waters). Les méthodes de chromatographies analytiques (HPLC et LCMS) sont décrites dans un paragraphe dédié. La pureté des intermédiaires de synthèse était déterminée par RMN ¹H ou par HPLC en phase inverse. La pureté des produits finaux était déterminée par HPLC en phase inverse et était confirmée > 95%.

Les composés suivants ont été synthétisés : 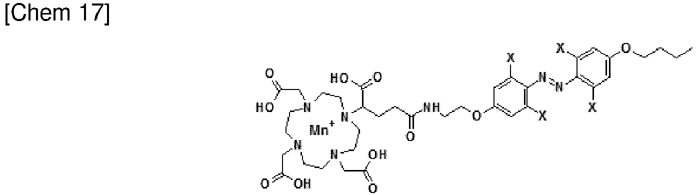
Avec X=H ou F,
Avec M=Cu, Ga, Y, In, Eu, Gd, Yb ou Bi,
Avec n=2 ou 3 selon le schéma de synthèse suivant

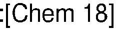

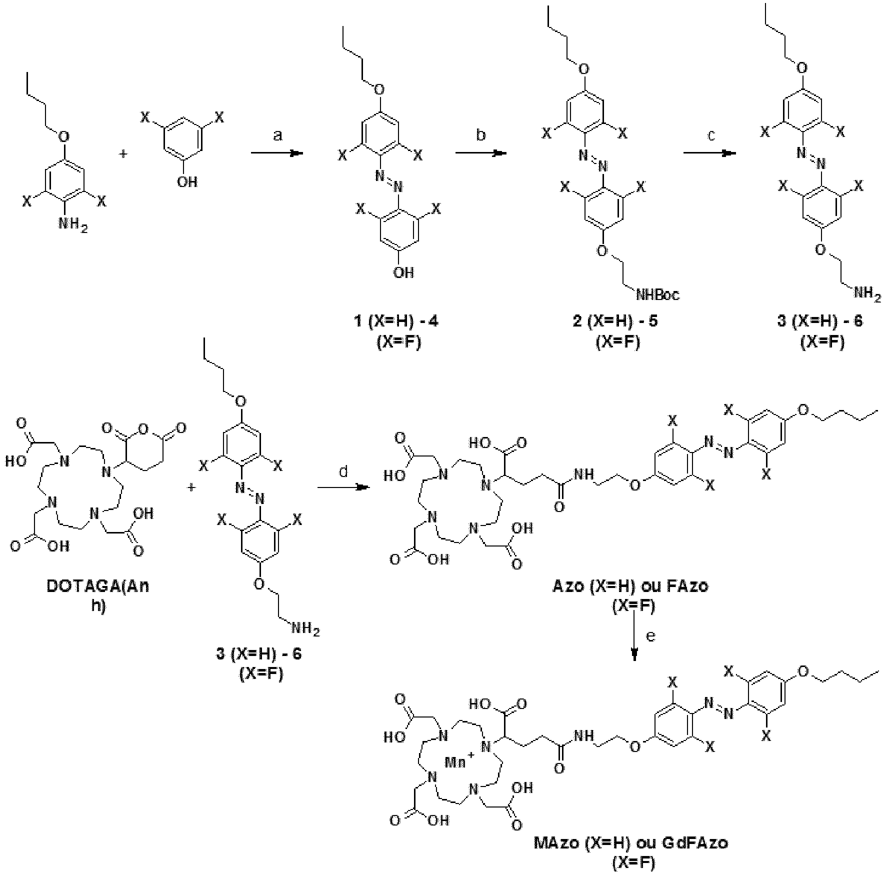

### Synthèse du 4-hydroxy-4'-butoxyazobenzène (1)

La 4-butoxyaniline (2,00 mL, 12,0 mmol) et le nitrite de sodium (0,854 g, 12,0 mmol, 1,0 équiv) étaient dissous dans un mélange EtOH/H₂O 1/1 (24 mL) et le milieu était refroidi à 0 °C. De la glace (12 g) était introduite dans le milieu avant l'addition prudente de HCl cc (2,6 mL). Une solution aqueuse (6,3 mL) de phénol (1,14 g, 12,0 mmol, 1,0 équiv) et de NaOH (0,960 g, 24,0 mmol, 2,0 équiv) préalablement préparée et refroidie à 0 °C était prudemment introduite dans le milieu à 0 °C. Le milieu était agité pendant 20 min à 0 °C puis pendant 70 min à ta. Après ajustement du pH à 1 (HCl cc), le milieu était laissé au repos pendant 30 min à ta avant filtration. Le précipité était lavé avec de l'eau (4x50 mL), solubilisé dans du DCM et la phase organique était séchée sur MgSO₄ avant d'être concentrée. Le 4-hydroxy-4'-butoxyazobenzène 1 (2,76 g, 10,2 mmol, 85%) était isolé sous la forme d'une poudre noire amorphe.

**RMN ¹H** (400 MHz; CDCl₃) δ (ppm) : 7,87 (d; *J* = 8,9 Hz; 2H); 7,82 (d; *J* = 8,8 Hz; 2H); 6,98 (d; *J* = 8,9 Hz; 2H); 6,91 (d; *J* = 8,8 Hz; 2H); 4,03 (t; *J* = 6,5 Hz; 2H); 1,85 - 1,75 (m; 2H); 1,58 - 1,45 (m; 2H); 0,99 (t; *J* = 7,4 Hz; 3H).

**RMN ¹³C** (75 MHz; CDCl₃) δ (ppm) : 161,51; 158,38; 146,89; 146,63; 124,81; 124,56; 116,07; 114,92; 68,22; 31,35; 19,33; 13,96.

**HRMS** (m/z): calculé pour C₁₆H₁₉N₂O₂, 271,1447 ([M+H]⁺). Trouvé 271,1440.

**R*_{f}*** = 0,36 (SiO₂; DCM; UV).

### Synthèse du 4-(N-(tert-butyloxycarbonyl)-éthoxyamine)-4'-butoxyazobenzène (2)

Le 4-hydroxy-4'-butoxyazobenzène **1** (2,00 g, 7,40 mmol) et le K₂CO₃ (1,53 g, 11,1 mmol, 1,5 équiv) étaient dissous dans l'acétone (25 mL). Après 30 min d'agitation à ta sous argon, le bromure de 2-(Boc-amino)éthyle (4,98 g, 22,2 mmol, 3,0 équiv) était introduit dans le milieu. Après agitation à reflux pendant 18 h, le milieu était filtré à chaud et le précipité était lavé avec de l'acétone chaud (75 mL). Le filtrat était laissé au repos à ta pendant 30 min avant filtration et le précipité 1 ainsi obtenu était lavé avec de l'acétone froid. Le filtrat était laissé au repos à 0 °C pendant 3 h avant filtration. Le précipité 2 obtenu était lavé avec de l'acétone froid et ajouté au précipité 1. Le filtrat était concentré et purifié par chromatographie flash sur silice (DCM). Le 4-(*N*-(*tert*-butyloxycarbonyl)-éthoxyamine)-4'-butoxyazobenzène **2** (2,27 g, 5,49 mmol, 74%) était isolé sous la forme d'une poudre jaune amorphe (57% par précipitation, 17% par chromatographie flash). **RMN ¹H** (400 MHz; CDCl₃) δ (ppm) : 7,86 (2d; *J* = 8,9 Hz; 4H); 6,99 (2d; *J* = 8,9 Hz; 4H); 4,10 (t; *J* = 5,0 Hz; 2H); 4,04 (t; *J* = 6,5 Hz; 2H); 3,57 (m; 2H); 1,87 - 1,74 (m; 2H); 1,64 - 1,40 (m; 11H); 1,00 (t; *J* = 7,4 Hz; 3H).

**RMN ¹³C** (100 MHz; CDCl₃) δ (ppm) : 161,46; 160,62; 156,03; 147,51; 147,07; 124,52; 124,48; 114,84; 114,82; 79,79; 68,18; 67,64; 40,27; 31,42; 28,55; 19,38; 13,98.

**HRMS** (m/z): calculé pour C₂₃H₃₂N₃O₄; 414,2393 ([M+H]⁺). Trouvé 414,2396.

**R*_{f}*** = 0,90 (SiO₂; DCM/MeOH 98:2; UV).

### Synthèse du 4-aminoéthoxy-4'-butoxyazobenzène (3)

Le 4-(*N*-(tert-butyloxycarbonyl)-éthoxyamine)-4'-butoxyazobenzène **2** (1,26 g, 3,04 mmol) était dissous dans un mélange DCM/TFA 4:1 (63 mL) et le milieu était agité pendant 1,5 h à ta. Après concentration, de l'éther diéthylique (250 mL) était introduit et le précipité formé était concrétisé pendant 1 h avant filtration et lavage à l'éther diéthylique (4x50 mL). Le 4-aminoéthoxy-4'-butoxyazobenzène **3** (1,25 g, 2,93 mmol, 96%, sel de TFA) était isolé sous la forme d'une poudre jaune amorphe.

**RMN ¹H** (400 MHz; MeOD) δ (ppm) : 7,88 (d; *J* = 8,9 Hz; 2H); 7,85 (d; *J* = 8,9 Hz; 2H); 7,15 (d; *J* = 8,9 Hz; 2H); 7,04 (d; *J* = 8,9 Hz; 2H); 4,32 (t; *J* = 4,4 Hz; 2H); 4,06 (t; *J* = 6,4 Hz; 2H); 3,41 (t; *J* = 4,4 Hz; 2H); 1,86 - 1,73 (m; 2H); 1,60 - 1,46 (m; 2H); 1,00 (t; *J* = 7,4 Hz; 3H).

**RMN ¹³C** (75 MHz; MeOD) δ (ppm) : 163,03; 161,34; 148,97; 148,10; 125,42; 125,30; 115,99; 115,80; 69,12; 65,59; 40,24; 32,43; 20,28; 14,16.

**HRMS** (m/z): calculé pour C₁₈H₂₄N₃O₂, 314,1869 ([M+H]⁺). Trouvé 314,1864.

**HPLC** (méthode A); t_{R} 17,92 min (cis)-20,35 min *(trans).*

**R*_{f}*** = 0,70 (SiO₂; DCM/MeOH 95:5; UV ou ninhydrine).

### Synthèse du 4-(N-(1,4,7,10-tétraazacyclododécane-1,4,7-tri-acide acétique-10-glutaryl)-éthoxyamine)-4'-butoxyazobenzène (Azo)

Le 4-aminoéthoxy-4'-butoxyazobenzène 3 (259 mg, 0,606 mmol, sel de TFA) était dissous dans la DMF anhydre (4,3 mL). Après introduction de la triéthylamine (253 µL, 1,81 mmol, 3,0 équiv), le milieu était agité pendant 5 min à ta sous argon. L'anhydride de DOTAGA (278 mg, 0,606 mmol, 1,0 équiv) était ensuite introduit et le milieu était agité pendant 21 h à 70 °C sous argon. Après concentration, de l'éther diéthylique (100 mL) était introduit et le précipité formé était concrétisé pendant 30 min avant filtration et lavage avec de l'éther diéthylique (2x50 mL). Le brut était purifié par chromatographie flash en phase inverse (RP-18, Biotage, gradient H₂O 0,05% FA/CH₃CN 0,05% FA 1:0 to 2:8). Le 4-(N-(1,4,7,10-tétraazacyclododécane-1,4,7-tri-acide acétique-10-glutaryl)-éthoxyamine)-4'-butoxyazobenzène **Azo** (294 mg, 0,381 mmol, 63%) était isolé par lyophilisation sous la forme d'une poudre jaune électrostatique.

**HRMS** (m/z): calculé pour C₃₇H₅₄N₇O₁₁; 772,3881 ([M+H]⁺). Trouvé 772,3883.

**LCMS** (méthode B); t_{R} 12,357 min (cis)-14,437 min *(trans).*

### Synthèse du 2,6-difluoro-4-hydroxy-2',6'-difluoro-4'-butoxyazobenzène (4)

La 2,6-difluoro-4-butoxyaniline (2,20 g, 10,9 mmol) et le nitrite de sodium (0,755 g, 10,9 mmol, 1,0 équiv) étaient dissous dans un mélange EtOH/H₂O 1/1 (22 mL) et le milieu était refroidi à 0 °C. De la glace (11 g) était introduite dans le milieu avant l'addition prudente de HCl cc (2,37 mL). Une solution aqueuse (5,7 mL) de 3,5-difluorophénol (1,42 g, 10,9 mmol, 1,0 équiv) et de NaOH (0,876 g, 21,9 mmol, 2,0 équiv) préalablement préparée et refroidie à 0 °C était prudemment introduite dans le milieu à 0 °C. Le milieu était agité pendant 20 min à 0 °C puis pendant 70 min à ta. Après ajustement du pH à 1 (HCl cc), le milieu était laissé au repos pendant 30 min à ta avant filtration. Le précipité était lavé avec de l'eau (4x50 mL), solubilisé dans du DCM et la phase organique était séchée sur MgSO₄ avant d'être concentrée. Le résidu (liquide visqueux noir) était concentré sous une rampe à vide (3,15 g) et était utilisé sans autre purification dans l'étape suivante.

**RMN ¹H** (400 MHz; DMSO) δ (ppm) : 6,93 (d; *J_{H-F}* = 11,4 Hz; 2H); 6,64 (d; *J_{H-F}* = 11,4 Hz; 2H); 4,09 (t; *J* = 6,5 Hz; 2H); 1,77 - 1,66 (m; 2H); 1,49 - 1,38 (m; 2H); 0,94 (t; *J* = 7,4 Hz; 3H).

**RMN ¹³C** (101 MHz; DMSO) δ (ppm) : 161,40 (t; *J_{C-F}* = 14,8 Hz); 161,11 (t; *J_{C-F}* = 14,8 Hz); 157,69 (dd; *J_{C-F}* = 39,1; 7,7 Hz); 155,13 (dd; *J_{C-F}* = 38,2; 7,8 Hz); 125,00 (t; *J_{C-F}* = 10,2 Hz); 124,05 (t; *J_{C-F}* = 10,3 Hz); 100,26 (dd; *J_{C-F}* = 22,6; 2,0 Hz); 99,68 (dd; *J_{C-F}* = 24,2; 2,1 Hz); 68,74 (s); 30,31 (s); 18,55 (s); 13,58 (s).

**RMN ¹⁹F** (376 MHz; DMSO; découplé) δ (ppm) : -118,99; -119,27.

**RMN ¹⁹F** (376 MHz; DMSO) δ (ppm) :-118,99 (d; *J_{F-H}* = 12,1 Hz); -119,27 (d; *J_{F-H}* = 12,0 Hz).

**HRMS** (m/z): calculé pour C₁₆H₁₅F₄N₂O₂, 343,1070 ([M+H]⁺). Trouvé 343,1060.

**R*_{f}*** = 0,20 (SiO₂; cyclohexane/acétate d'éthyle 9:1; UV).

### Synthèse du 2,6-difluoro-4-(N-(tert-butyloxycarbonyl)-éthoxyamine)-2',6'-difluoro-4'-butoxyazobenzène (5)

Le 2,6-difluoro-4-hydroxy-2',6'-difluoro-4'-butoxyazobenzène **4** (2,18 g du brut, 6,37 mmol considéré) et le K₂CO₃ (1,32 g, 9,57 mmol, 1,5 équiv) étaient dissous dans l'acétone (22 mL). Après 30 min d'agitation à ta sous argon, le bromure de 2-(Boc-amino)éthyle (4,29 g, 19,1 mmol, 3,0 équiv) était introduit dans le milieu. Après agitation à reflux pendant 18 h, le milieu était concentré et purifié par chromatographie flash sur silice (cyclohexane/acétate d'éthyle, gradient 1/0 à 7/3). Le 2,6-difluoro-4-(*N*-(*tert-*butyloxycarbonyl)-éthoxyamine)-2',6'-difluoro-4'-butoxyazobenzène **5** (624 mg, 1,28 mmol, 17% sur 2 étapes) était isolé sous la forme d'une poudre jaune amorphe.

**RMN ¹H** (400 MHz; DMSO) δ (ppm) : 7,04 (s; 1H); 6,95 (d; *J_{H-F}* = 11,8 Hz; 4H); 4,10 (m; 4H); 3,34 - 3,26 (m; 2H); 1,75 - 1,67 (m; 2H); 1,49-1,39 (m; 2H); 1,38 (s; 9H); 0,94 (t; *J* = 7,4 Hz; 3H).

**RMN ¹³C** (101 MHz; DMSO) δ (ppm) : 161,53 (t; *J_{c-F}* = 14,3 Hz); 161,16 (t; *J_{c-F}* = 14,2 Hz); 157,59 (t; *J_{C-F}* = 7,9 Hz); 155,64 (s); 155,03 (t; *J_{C-F}* = 7,7 Hz); 125,09 (t; *J_{C-F}* = 8,3 Hz); 124,90 (t; *J_{c-F}* = 8,3 Hz); 99,90 (dd; *J_{C-F}* = 11,3; 1,7 Hz); 99,67 (dd; *J_{C-F}* = 11,3; 1,7 Hz); 77,86 (s); 68,80 (s); 67,87 (s); 38,71 (s); 30,29 (s); 28,17 (s); 18,53 (s); 13,57 (s).

**RMN ¹⁹F** (376 MHz; DMSO; découplé) δ (ppm) : -118,79; -118,85.

**RMN ¹⁹F** (376 MHz; DMSO) *δ* (ppm) : -118,79 (d; *J_{F-H}* = 12,0 Hz); -118,85 (d; *J_{F-H}* = 11,9 Hz).

**HRMS** (m/z): calculé pour C₂₃H₂₈F₄N₃O₄; 486,2016 ([M+H]⁺). Trouvé 486,2015.

**R*_{f}*** = 0,30 (SiO₂; cyclohexane/acétate d'éthyle 8:2; UV).

### Synthèse du 2,6-difluoro-4-aminoéthoxy-2',6'-difluoro-4'-butoxyazobenzène (6)

Le 2,6-difluoro-4-(*N*-(*tert-*butyloxycarbonyl)-éthoxyamine)-2',6'-difluoro-4'-butoxyazobenzène **5** (879 mg, 1,81 mmol) était dissous dans un mélange DCM/TFA 4:1 (37 mL) et le milieu était agité pendant 1,5 h à ta. Après concentration, de l'éther diéthylique (200 mL) était introduit et le précipité 1 formé était concrétisé pendant 1 h avant filtration et lavage à l'éther diéthylique (4x100 mL). Le filtrat était concentré et un précipité visqueux 2 était formé dans le n-hexane (200 mL), filtré et lavé avec du n-hexane (3x200 mL). Les précipités 1 et 2 étaient séchés sous une rampe à vide. Le 2,6-difluoro-4-aminoéthoxy-2',6'-difluoro-4'-butoxyazobenzène **6** (858 mg, 1,72 mmol, 95%, sel de TFA) était isolé sous la forme d'une poudre jaune amorphe (précipité 1, 70%) et d'une huile jaune (précipité 2, 25%).

**RMN ¹H** (400 MHz; DMSO) δ (ppm) : 7,98 (s; 3H); 7,00 (d; *J_{H-F}* = 11,3 Hz; 2H); 6,96 (d; *J_{H- F}* = 11,8 Hz; 2H); 4,30 (t; *J* = 5,0 Hz; 2H); 4,11 (t; *J* = 6,5 Hz; 2H); 3,26 (t; *J* = 5,0 Hz; 2H); 1,80 - 1,63 (m; 2H); 1,53 - 1,33 (m; 2H); 0,94 (t; *J* = 7,4 Hz; 3H).

**RMN ¹³C** (101 MHz; DMSO) δ (ppm) : 161,76 (t; *J_{C-F}* = 14,4 Hz); 160,27 (t; *J_{C-F}* = 14,0 Hz); 157,57 (dd; *J_{C-F}* = 25,8; 7,6 Hz); 155,00 (dd; *J_{C-F}* = 25,9; 7,4 Hz); 125,57 (s); 124,86 (s); 100,07 (dd; *J_{C-F}* = 24,7; 2,7 Hz); 99,80 (dd; *J_{C-F}* = 24,7; 2,5 Hz); 68,85 (s); 65,82 (s); 38,06 (s); 30,29 (s); 18,54 (s); 13,57 (s).

**RMN ¹⁹F** (376 MHz; DMSO; découplé) δ (ppm) : -73,46; -118,60; -118,74.

**RMN ¹⁹F** (376 MHz; DMSO) δ (ppm) : -73,46 (s); -118,60 (d; *J_{F-H}* = 11,9 Hz); -118,73 (d; *J_{F-H}* = 11,8 Hz).

**HRMS** (m/z): calculé pour C₁₈H₂₀F₄N₃O₂; 386,1492 ([M+H]⁺). Trouvé 386,1491.

**R*_{f}*** = 0,33 (SiO₂; DCM/MeOH 95:5; UV ou ninhydrine).

### Synthèse du 2,6-difluoro-4-(N-(1,4,7,10-tétraazacyclododécane-1,4,7-tri-acide acétique-10-glutaryl)-éthoxyamine)-2',6'-difluoro-4'-butoxyazobenzène (FAzo)

Le 2,6-difluoro-4-aminoéthoxy-2',6'-difluoro-4'-butoxyazobenzène 6 (267 mg, 0,535 mmol, sel de TFA) était dissous dans la DMF anhydre (3,8 mL). Après introduction de la triéthylamine (223 µL, 1,60 mmol, 3,0 équiv), le milieu était agité pendant 5 min à ta sous argon. L'anhydride de DOTAGA (245 mg, 0,533 mmol, 1,0 équiv) était ensuite introduit et le milieu était agité pendant 21 h à 70 °C sous argon. Après concentration, de l'éther diéthylique (100 mL) était introduit et le précipité formé était concrétisé pendant 30 min avant filtration et lavage avec de l'éther diéthylique (2x50 mL). Le brut était purifié par chromatographie flash en phase inverse (RP-18, Biotage, gradient H₂O 0,05% FA/CH₃CN 0,05% FA 1:0 to 2:8). Le 2,6-difluoro-4-(*N*-(1,4,7,10-tétraazacyclododécane-1,4,7-tri-acide acétique-10-glutaryl)-éthoxyamine)-2',6'-difluoro-4'-butoxyazobenzène **FAzo** (92,1 mg, 0,109 mmol, 20%) était isolé par lyophilisation sous la forme d'une poudre jaune électrostatique.

**HRMS** (m/z): calculé pour C₃₇H₅₀F₄N₇O₁₁; 844,3504 ([M+H]⁺). Trouvé 844,3495.

**LCMS** (méthode B); t_{R} 14,107 min (*cis*)-14,894 min (*trans*).

### Protocole général pour la complexation de Azo et FAzo

**Azo** ou **FAzo** et le réactif métallique étaient dissous dans H2O (26 mM). Après ajustement du pH à 5,5, le milieu était agité pendant 17 h à 50 °C en contrôlant à ce que le pH reste dans la gamme 5,5-6,0. Le pH était ensuite ajusté à 6,5 avant concentration du milieu par lyophilisation puis purification par chromatographie flash en phase inverse (RP-18, Biotage, gradient H2O/CH3CN 1:0 à 0:1. Le produit final était isolé par lyophilisation sous la forme d'une poudre électrostatique.

### [Table 1]

**Tableau 1. Conditions expérimentales des réactions de complexation.**

| Métal | Produit de départ | Echelle de réaction (µmol produit de départ) | Type de réactif | Quantité de réactif (équiv) | Rendement (%) | Couleur du produit final |
|---|---|---|---|---|---|---|
| Cu | Azo | 104 | Cu(OAc)₂ | 1,05 | 60 | vert |
| Ga | Azo | 78,0 | GaNO₃ | 2,05 | 82 | jaune |
| Y | Azo | 90,0 | Y Cl₃₋6H₂O | 1,05 | 31 | jaune |
| In | Azo | 80,0 | InCl₃₋4H₂O | 1,05 | 27 | jaune |
| Eu | Azo | 130 | EuCl₃₋6H₂O | 1,05 | 39 | jaune |
| Gd | Azo | 259 | GdCl₃₋6H₂O | 1,05 | 88 | jaune |
| Gd | FAzo | 142 | GdCl₃₋6H₂O | 1,05 | 97 | jaune |
| Yb | Azo | 110 | YbCl₃₋6H₂O | 1,05 | 21 | jaune |
| Bi | Azo | 104 | BiCl₃* | 1,05 | 73 | jaune |

| | | | | | | |
|---|---|---|---|---|---|---|
| *: 250 mM dans HCL 6N. | | | | | | |

### Copper 4-(N-(1,4,7,10-tétraazacyclododécane-1,4,7-tri-acide acétique-10-glutaryl)-éthoxyamine)-4'-butoxyazobenzène (CuAzo)

**HRMS** (m/z): calculé pour C₃₇H₅₁N₇NaO₁₁Cu; 855,2840 ([M+Na]⁺). Trouvé 855,2854. **LCMS** (méthode B); t_{R} 13,550 min (cis)-15,934 min (*trans*).

### Gallium 4-(N-(1,4,7,10-tétraazacyclododécane-1,4,7-tri-acide acétique-10-glutaryl)-éthoxyamine)-4'-butoxyazobenzène (GaAzo)

**HRMS** (m/z): calculé pour C₃₇H₅₁N₇O₁₁Ga; 838,2902 ([M+H]⁺). Trouvé 838,2906.

**LCMS** (méthode B); t_{R} 12,840 min (cis)-15,072 min *(trans).*

### Yttrium 4-(N-(1,4,7,10-tétraazacyclododécane-1,4,7-tri-acide acétique-10-glutaryl)-éthoxyamine)-4'-butoxyazobenzène (YAzo)

**HRMS** (m/z): calculé pour C₃₇H₄₉N₇Na₂O₁₁Y; 902,2344 ([M-H+2Na]⁺). Trouvé 902,2349. **LCMS** (méthode B); t_{R} 15,957 min (*cis*)-19,660 min *(trans).*

### Indium 4-(N-(1,4,7,10-tétraazacyclododécane-1,4,7-tri-acide acétique-10-glutaryl)-éthoxyamine)-4'-butoxyazobenzène (InAzo)

**HRMS** (m/z): calculé pour C₃₇H₄₉N₇Na₂O₁₁In; 928,2324 ([M-H+2Na]⁺). Trouvé 928,2319. **LCMS** (méthode B); t_{R} 15,451 min (cis)-18,899 min *(trans).*

### Europium 4-(N-(1,4,7,10-tétraazacyclododécane-1,4,7-tri-acide acétique-10-glutaryl)-éthoxyamine)-4'-butoxyazobenzène (EuAzo)

**HRMS** (m/z) (mode négatif): calculé pour C₃₇H₄₉N₇O₁₁Eu; 920,2702 ([M-H]). Trouvé 920,2694.

**LCMS** (méthode B); t_{R} 13,419 min (*cis*)-16,718 min *(trans).*

### Gadolinium 4-(N-(1,4,7,10-tétraazacyclododécane-1,4,7-tri-acide acétique-10-glutaryl)-éthoxyamine)-4'-butoxyazobenzène (GdAzo)

**HRMS** (m/z): calculé pour C₃₇H₄₉N₇Na₂O₁₁Gd; 971,2527 ([M-H+2Na]⁺). Trouvé 971,2529.

**LCMS** (méthode B); t_{R} 15,956 min (*cis*)-19,633 min *(trans).*

### Gadolinium 2,6-difluoro-4-(N-(1,4,7,10-tétraazacyclododécane-1,4,7-tri-acide

**acétique-10-glutaryl)-éthoxyamine)-2',6'-difluoro-4'-butoxyazobenzène (GdFAzo)**

**HRMS** (m/z): calculé pour C₃₇H₄₅F₄N₇Na₂O₁₁Gd; 1043,2150 ([M-H+2Na]⁺). Trouvé 1043,2158.

**LCMS** (méthode B); t_{R} 18,900 min (cis)-20,524 min *(trans).*

### Ytterbium 4-(N-(1,4,7,10-tétraazacyclododécane-1,4,7-tri-acide acétique-10-glutaryl)-éthoxyamine)-4'-butoxyazobenzène (YbAzo)

**HRMS** (m/z): calculé pour C₃₇H₄₉N₇Na₂O₁₁Yb; 987,2674 ([M-H+2Na]⁺). Trouvé 987,2683.

**LCMS** (méthode B); t_{R} 16,112 min (cis)-19,968 min *(trans).*

### Bismuth 4-(N-(1,4,7,10-tétraazacyclododécane-1,4,7-tri-acide acétique-10-glutaryl)-éthoxyamine)-4'-butoxyazobenzène (BiAzo)

**HRMS** (m/z): calculé pour C₃₇H₄₉N₇Na₂O₁₁Bi; 1022,3089 ([M-H+2Na]⁺). Trouvé 1022,3093.

**LCMS** (méthode B); t_{R} 14,235 min (cis)-19,055 min *(trans).*

### III. Expériences d'irradiations ionisantes et sur cellules

### Général

Les milieux tampons tels que le *phosphate buffered saline* (PBS), *Dulbecco's Modified Eagle Medium-high glucose* (DMEM), *Roswell Park Memorial Institute medium* (RPMI), la pénicilline, la streptomycine et la solution de bleu de trypan (0.4%) ont été achetés chez Sigma Aldrich (France). Le sérum de foetus bovin (FBS) a été acheté chez Gibco (France), l'iodure de propidium (IP) a été acheté chez Thermo Fisher Scientific (France) et l'hydrochlorure de gemcitabine (Gem) a été acheté chez Sequoia Research Products (UK).

### Lignées cellulaires

Les cellules du cancer de pancréas humain (PANC-1) et de leucémie lymphoblastique aiguë humaine (CCRF-CEM) ont été achetées chez ATCC (US) et maintenues comme recommandé par le fournisseur. Les cellules de leucémie lymphoblastique aiguë humaine résistantes à la Gem (CCRF-CEM ARAC 8C, récepteur hENT-1 non exprimé) ont été gracieusement fournies par Dr. Buddy Ullmann (Oregon Health Sciences University). Brièvement, les cellules PANC-1 étaient maintenues dans le tampon DMEM complété avec 10% (v/v) de FBS inactivé par chauffage. Les cellules CCRF-CEM et CCRF-CEM ARAC-8C étaient maintenues dans le tampon RPMI complété avec 10% (v/v) de FBS inactivé par chauffage. Tous les milieux cellulaires étaient complétés avec de la pénicilline (50 U.mL⁻¹) et de la streptomycine (0.05 mg.mL⁻¹). Les cellules étaient maintenues dans une atmosphère humide à 37 °C avec 5% de CO2. Les cellules étaient utilisées avant d'atteindre le dix-huitième passage et étaient récoltées à une confluence de 70-80%.

### Sources d'irradiations ionisantes

**Irradiation UV.** L'irradiation UV pour induire l'isomérisation de l'isomère *trans* en isomère *cis* était effectuée dans une chambre CN-15.LC (Vilber Lourmat) équipée avec deux tubes de 15W (365 nm) qui fournissait une irradiance de 0,817 mW.cm⁻² à la position d'irradiation (déterminé par un radiomètre à microprocesseur contrôlé Cole-Parmer VLX-3W calibré pour 365 nm, Vilber Lourmat).

**Irradiation par rayons-X.** L'irradiation par rayons-X était effectuée avec un générateur de rayons-X (Xrad 320 Dx) fournissant des photons à une énergie moyenne de 80 keV (gamme de 0-200 keV) à un débit de dose d'environ 1 Gy/min (utilisation à 200 kV, 20 mA). Les doses d'irradiation sont exprimées en Gray.

**Irradiation par rayons-gamma.** L'irradiation par rayons-gamma était effectuée avec une source de Cesium-137 (IBL 637) fournissant des photons de 662 keV à un débit de dose d'environ 1 Gy/min. Les doses d'irradiation sont exprimées en Gray (1 Gy=1 J/L). **Irradiation par faisceau d'électrons.** L'irradiation par faisceau d'électrons était effectuée avec un accélérateur linéaire d'électrons (Linac, Kinetron) fournissant des électrons à une énergie de 4,5 MeV à un débit de dose d'environ 4 Gy/min. Les doses d'irradiation sont exprimées en Gray.

### Quantification de l'isomérisation induite par rayonnements ionisants par spectrophotométrie d'absorbance et HPLC (Figure 1)

Les composés contenant du Gd (GdAzo ou GdFAzo) et les composés contrôles (Azo ou FAzo) (50 µM, 200 µL, PBS) étaient introduits dans deux microplaques 96 puits (10 puits pour chaque composé). Les deux microplaques étaient tout d'abord irradiées sous UV (365 nm, 0,817 mW.cm⁻², 5 min). Une microplaque (plaque 1) était ensuite gardée à l'obscurité et utilisée comme contrôle non irradié alors que la seconde (plaque 2) était irradiée par un incrément de doses d'irradiations ionisantes (2 Gy, 3 Gy, 5 Gy et 10 Gy). Après chaque irradiation, des analyses par spectrophotométrie d'absorbance et par injection en HPLC (méthode C) étaient effectuées sur les composés non irradiés (plaque 1) et irradiés (plaque 2). Un décalage de 26 min entre l'irradiation par UV des plaques 1 et 2 était effectué pour permettre l'analyse des composés contrôles non irradiés (plaque 1) au même moment que les composés irradiés par rayonnements ionisants (plaque 2) après irradiation UV. L'expérience complète était réalisée en 4,6 h. Les tracés des spectres d'absorbance étaient obtenus par la moyenne de triplicata. La quantité relative de chaque isomère était obtenue par HPLC (détection à la longueur d'onde du point isobestique dans les conditions d'élution) et l'activation moléculaire (%) était déterminée par la différence de proportion en isomère *trans* dans le milieu (3 expériences indépendantes).

### Microscopie confocale sans rayonnements ionisants (Figure 2a)

Les cellules (PANC-1) étaient transférées dans une plaque d'imagerie (micro-slide 8 well ibiTreat achetée chez Ibidi, Allemagne) 24 h avant le début de l'expérience, et étaient maintenues dans le milieu de culture (33000 cellules dans 200 µL/puits) sous atmosphère humide à 37 °C avec 5% de CO2. Juste avant l'expérience, le milieu cellulaire était remplacé par du milieu de culture sans FBS et sans rouge phénol (100 µL) puis du IP (5 µL, concentration finale 1 µM) était ajouté dans le milieu. 15 min après l'ajout du IP, une première série d'images était acquise. Puis le composé cis-GdAzo ou trans-GdAzo (100 µL, concentration finale 0 µM, 250 µM ou 500 µM) était introduit dans le milieu cellulaire. La plaque d'imagerie était ensuite gardée à l'obscurité et des images étaient acquises toutes les 5 min pendant 1 h. Le composé cis-GdAzo était obtenu par irradiation UV (365 nm, 0,817 mW.cm⁻², 30 min) du composé trans-GdAzo (105 µL, 500 µM ou 1 mM, *cis-*GdAzo > 85%) dans une microplaque 96 puits. Les cellules étaient observées avec un microscope inversé Leica TCS SP8 gated-STED (Leica, Allemagne) en utilisant un objectif HC PL Fluotar CS2 10x/0.30 sec. L'instrument était équipé avec un laser à lumière blanche (longueur d'onde d'excitation 538 nm). L'émission de fluorescence rouge était collectée sur une bande passante de 560-650 nm et les images en transmission étaient obtenues avec la même ligne de lumière et un détecteur PMT-trans. Le sténopé était réglé à 1,0 Airy (73.19 µm de diamètre). Les images numériques 16 bits ont été obtenues avec le logiciel Leica SP8 LAS X (version 2.0.1, Leica, Allemangne).

### Microscopie confocale en présence de rayonnements ionisants (Figures 2b-c)

Les cellules (PANC-1) étaient transférées dans une plaque d'imagerie (Lab-Tek chamber slide system, glass, 8 well achetée chez Thermo Fisher Scientific, France) 24 h avant le début de l'expérience, et étaient maintenues dans le milieu de culture (10000 cellules dans 200 µL/puits) sous atmosphère humide à 37 °C avec 5% de CO2. Juste avant l'expérience, le milieu cellulaire était remplacé par du PBS (100 µL) et du IP (5 µL, concentration finale 1 µM) était ajouté dans le milieu. 15 min après l'ajout du IP, *cis-*GdAzo (100 µL, concentration finale 0 µM, 250 µM, 500 µM ou 850 µM) était introduit dans le milieu cellulaire. Une première série d'images était acquise à ce stade, puis la plaque d'imagerie était irradiée (rayons-gamma, 2 Gy). La plaque d'imagerie était ensuite gardée à l'obscurité à 37 °C (chauffe-platine) et des images étaient acquises toutes les 5 min pendant 30 min, puis toutes les 10 min pendant 90 min. Une procédure similaire était utilisée pour l'expérience contrôle sans irradiation excepté que la plaque d'imagerie n'était pas irradiée par les rayons-gamma. Le composé cis-GdAzo était obtenu par irradiation UV (365 nm, 0,817 mW.cm⁻², 30 min) du composé trans-GdAzo (105 µL, 500 µM, 1 mM ou 1,7 mM, cis-GdAzo > 85%) dans une microplaque 96 puits. Les cellules étaient observées avec un microscope inversé Nikon (Nikon Instruments Inc., Tokyo, Japon) en utilisant un objectif x10 sec, N.A. 0,4 et équipé avec une tête CSU-X1 Yokogawa. L'instrument était équipé avec une diode laser à 561 nm comme longueur d'onde d'excitation. L'émission de fluorescence rouge était collectée sur une bande passante de 598-672 nm et les images en transmission étaient obtenues avec une diode blanche. Le sténopé était réglé à 50 µm et la lentille de grossissement à 1,2. Les images étaient enregistrées par une caméra e-Volve s-CMOS (Photometrics). Quatre images étaient enregistrées par puits et deux puits étaient utilisés pour chaque concentration de cis-GdAzo. Les analyses des images numériques 16 bits étaient effectuées sur le logiciel ImageJ (version 1.50i complétée avec le plugin Adjustable Watershed). Le nombre de cellules sur les images acquises avant irradiation était déterminé manuellement et le nombre de cellules fluorescentes sur les images acquises avant et après irradiation était calculé automatiquement par l'utilisation d'un script codé sur ImageJ. La perméabilisation cellulaire était déterminée par la différence de proportion de cellules fluorescentes avant et 30 min après irradiation par rayons-gamma (3 expériences indépendantes).

### Effet thérapeutique sous rayonnements ionisants (Figures 2d-f)

Juste avant l'expérience, les cellules (CCRF-CEM ARAC-8C) étaient dispersées dans du PBS et transférées dans une microplaque de 48 puits (TPP cell culture microplates achetée chez Thermo Fisher Scientific, France) (40000 cellules dans 80 µL/puits). La Gem (20 µL, concentration finale 0,1 µM) ou le PBS (20 µL) et le composé cis-GdAzo (100 µL, concentration finale 0 µM, 250 µM, 500 µM ou 850 µM) étaient ajoutés juste avant l'irradiation du milieu par rayons-gamma (2 Gy). Le milieu était maintenu dans l'obscurité et une atmosphère humide à 37 °C avec 5% de CO2 pendant 1 h. Ensuite, du milieu de culture (600 µL) était ajouté dans chaque puits et les cellules étaient lavées par trois cycles de centrifugation (300 G, 5 min, 800 µL de milieu de culture utilisés pour chaque lavage). Les cellules étaient finalement dispersées dans du milieu de culture (600 µL) contenant ou non de la Gem (concentration finale 0 µM ou 0,1 µM) et étaient maintenues en atmosphère humide à 37 °C avec 5% de CO2 pendant 4 jours. Le nombre de cellules vivantes était déterminé par comptage cellulaire en présence de bleu de trypan 1/1 (v/v) (triplicata). L'expérience a été répétée trois fois de manière indépendante. La viabilité cellulaire était exprimée par le ratio du nombre de cellules vivantes après traitement sur le nombre de cellules vivantes sans aucun traitement (sans irradiation, en absence de Gem et de *cis*-GdAzo). Une procédure similaire était utilisée pour l'expérience contrôle sans irradiation excepté que la microplaque 48 puits n'était pas irradiée par les rayons-gamma. Une procédure similaire était utilisée pour l'expérience contrôle avec le Dotarem^{®} (Figure 2f) excepté que le Dotarem^{®} était utilisé à la place du cis-GdAzo. Le composé *cis*₋GdAzo était obtenu par irradiation UV (365 nm, 0,817 mW.cm⁻², 30 min) du composé *trans*-GdAzo (105 µL, 500 µM, 1 mM ou 1,7 mM, *cis*-GdAzo > 85%) dans une microplaque 96 puits.

Ces résultats démontrent :
(i) un nouveau concept d'activation de molécules thérapeutiques par l'utilisation de rayonnements ionisants (rayons X, rayons gamma, électrons) (Fig. 1a-f). Ces rayonnements ayant un pouvoir de pénétration dans les tissus vivants très important, ce nouveau concept d'activation a donc un fort potentiel pour une utilisation en clinique. L'ensemble des molécules organiques décrites aujourd'hui dans la littérature nécessite l'utilisation de rayonnements de l'UV au proche-IR, rayons qui ne pénètrent pas les tissus biologiques en profondeur (ils pénètrent à quelques centaines de micromètres dans les meilleures conditions).
(ii) qu'il est possible d'activer les composés MAzo (M=Cu, Ga, Y, In, Eu, Gd, Yb, Bi) par rayonnements ionisants avec des efficacités différentes en fonction du métal et de la source d'irradiation utilisés (Fig. 1a-c,f). Le composé GdFAzo peut également être activé par rayonnements ionisants (Fig. 1c-e). Ce composé est caractérisé par une relaxation thermique suffisamment lente pour envisager son étude *in vivo.* Bien que l'activation du composé GdFAzo soit relativement faible dans les conditions expérimentales utilisées *in vitro* (en comparaison au composé GdAzo, Fig. 1c), il est difficile de prévoir l'efficacité d'activation moléculaire qu'aurait le composé *in vivo.*
(iii) le type de métal qu'il faut utiliser pour permettre l'activation du motif organique. Il a été démontré que le nombre atomique Z du métal doit être supérieur à 39 (celui de l'Yttrium) pour permettre une activation conséquente (> 30% sous 5 Gy). En effet, 6 composés contenant un métal de taille supérieure ou égale à celle de l'Yttrium ont montré une telle activation alors que 2 composés contenant des métaux de tailles plus petites (Cu, Z=29, et Ga, Z=31) ont montré une faible activation (< 10% sous 5 Gy) (Fig. 1f).
(iv) que l'activation moléculaire peut être effectuée sous divers types de rayonnements ionisants (rayons X, rayons gamma, électrons), avec des particules différentes (photons et électrons) et des énergies variées (de 1 keV à 4,5 MeV) (Fig. 1c), et donc que l'ensemble des dispositifs de radiothérapie actuellement utilisés en clinique devrait être efficace pour engendrer l'activation moléculaire décrite.
(v) que le composé GdAzo permet d'induire la perméabilisation des membranes de cellules cancéreuses sous rayonnements ionisants à faible dose (6,9% (vs 1,2% sans irradiation), 8,2% (vs 2,8% sans irradiation) et 12,4% (vs 7,5% sans irradiation) aux concentrations en GdAzo de 250 µM, 500 µM et 850 µM respectivement par rayons gamma à 2 Gy) (Fig. 2a,b,c).
(vi) que le composé GdAzo permet d'induire une toxicité sur une lignée de cellules cancéreuses résistantes à la gemcitabine (leucémie lymphoblastique aiguë humaine), en l'absence ou en présence de gemcitabine (Fig. 2d,e), après traitement par rayons gamma à 2 Gy. En l'absence de gemcitabine, la viabilité cellulaire 4 jours après traitement est réduite à 23% (*vs* 79% sans irradiation), 18% (*vs* 42% sans irradiation) et 3,0% (vs 27% sans irradiation) aux concentrations en GdAzo de 250 µM, 500 µM et 850 µM respectivement. En présence de gemcitabine, la viabilité cellulaire 4 jours après traitement est réduite à 15% (vs 49% sans irradiation), 9,1% (vs 30% sans irradiation) et 1,2% (vs 16% sans irradiation) aux concentrations en GdAzo de 250 µM, 500 µM et 850 µM respectivement. Cette étude démontre que le composé est actif seul sous rayonnements ionisants et que la présence d'une substance active environnante ne favorise que légèrement l'effet thérapeutique du composé (Fig. 2d,e).
(vii) que la présence du motif azobenzène ou stillbène sur la molécule est nécessaire pour engendrer l'effet thérapeutique puisque le composé témoin commercial Dotarem^{®} (chélate de gadolinium seul) n'a aucun effet thérapeutique sous rayonnements ionisants (Fig. 2f).

## Revendications

1. Composé de formule (I) : 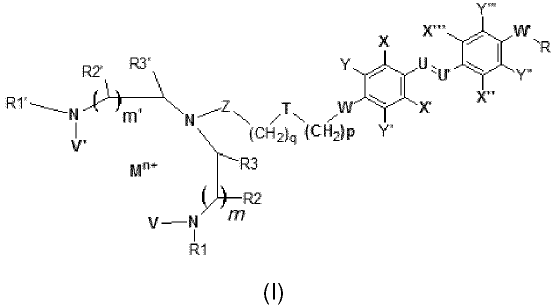 Dans laquelle :
M représente un atome de métal choisi parmi Ce(III), Pr(III), Nd(III), Sm(III), Eu(III), Gd(III), Tb(III), Dy(III), Ho(III), Er(III), Tm(III), Yb(III), Mg(ll), Ca(ll), Mn(II), Fe(Il), Fe(III), Cu(II), Zn(ll), Ga(III), Y(III), Zr(III), Tc(IV), Tc (VI), Tc (VII), Ru(ll), Ru(III), Ru(IV), Pd(ll), Ag(I), In(III), Hf (IV), Re(VI), W(ll), W(III), W(IV), W(V), W(VI), Os(III), Os(IV), Ir(III), Ir(IV), Pt(ll), Au(I), Au (III), TI(III), Zr(IV), Nb(III), Bi(lll) ;
n est égal à 1, 2, 3, 4, 5, 6 ou 7 ;
V et V' identiques ou différents sont des atomes d'hydrogène ou des chaînes alkoxy ou alkyle en C1-C10 linéaires ou ramifiées, ou des chaines alkyle en C1-C10 liées ensemble et comprenant un ou plusieurs hétéroatomes choisis parmi N, O ou S, optionnellement substituées par un ou plusieurs substituants choisis indépendamment parmi les atomes d'halogène, et les groupes nitrile, nitro, thio, amino, amido, aryle, hétéroaryle, hydroxyle, acide carboxylique ou carboxylate, pour former un cycle ;
R1, R1', R2, R2' identiques ou différents sont des atomes d'hydrogène, ou des chaines alkoxy ou alkyle en C1-C10 linéaires ou ramifiées éventuellement substituées par un ou plusieurs substituants choisis indépendamment parmi les atomes d'halogène, et les groupes nitrile, nitro, thio, amino, amido, aryle, hétéroaryle, hydroxyle, acide carboxylique ou carboxylate;
R3 et R3' identiques ou différents sont des atomes d'hydrogène ou des chaines alkoxy ou alkyle en C1-C10 linéaires ou ramifiées éventuellement substituées par un ou plusieurs substituants choisis indépendamment parmi les atomes d'halogène, et les groupes nitrile, nitro, thio, amino, amido, aryle, hétéroaryle, hydroxyle, acide carboxylique ou carboxylate, ou R3 et R3' sont liés ensemble pour former un hétérocycle ou hétéroaryle de 5 à 14 chainons ;
m et m' identiques ou différents sont égaux à 1 ou 2 ;
X, X', X", X‴, Y, Y', Y", Y‴ identiques ou différents sont choisis indépendamment parmi H ; les atomes d'halogène ; les groupes alkoxy, alkyle ou cycloalkyle éventuellement interrompus ou substitués par un ou plusieurs hétéroatome(s) ou groupe COOH, CONH₂ , COSH, OH, NH₂, SH ; les groupes aryle ou hétéroaryle de 5 à 12 chaînons éventuellement substitués par un ou plusieurs groupe COOH, CONH₂, COSH ; les groupes COOH ou NH₂ ;
Z représente un groupe alkyle éventuellement interrompu ou substitué par un ou plusieurs hétéroatome(s) ou groupe COOH, CONH₂ , COSH, OH, NH₂, SH ; un groupes aryle ou hétéroaryle de 5 à 12 chaînons éventuellement substitué par un ou plusieurs groupe COOH, CONH₂, COSH ; ou un groupe COOH ou NH₂ ;
W et W' identiques ou différents représentent indépendamment un groupe CH₂; ou un groupe aryle ou cylcoalkyle ; un atome d'oxygène ou d'azote (secondaire ou ternaire) ; une liaison amide ; une liaison ester ; une liaison thioéther ;
U et U' identiques ou différents représentent le groupe CH ou NH, étant entendu que la double liaison U=U' est sous forme *cis* ou *trans ;*
T représente un groupe CH₂; un groupe -C(=O)NH ; un groupe alkoxy, alkyle ou cycloalkyl éventuellement interrompu ou substitué par un ou plusieurs hétéroatome(s) ou groupe COOH, CONH₂, COSH, OH, NH₂, SH; un groupe aryle ou hétéroaryle de 5 à 12 chainons contenant un ou plusieurs hétéroatome(s) et/ou éventuellement substitué par un ou plusieurs groupes choisis parmi COOalkyle, CONHalkyle, COSHalkyle ;
R représente H, ou chaine alkoxy ou alkyle en C1-C18 linéaire ou ramifiée, éventuellement substituée par un ou plusieurs substituants choisis indépendamment parmi les atomes d'halogène, et les groupes nitrile, nitro, thio, amino, amido, aryle, hétéroaryle, hydroxyle, acide carboxylique ou carboxylate ;
p et q sont des entiers compris entre 0 et 6, notamment entre 1 et 6 ;
Etant entendu que pour assurer la neutralité de la molécule, les n charges cationiques de M peuvent être neutralisées par de 0 à n groupes carboxylate (-COOH) éventuellement substitués sur R1, R1', R2, R2', R3, R3', V, V', et/ou de 0 à n des contre-ions présents en solution le cas échéant ;
Sous forme d'isomère *cis* et/ou *trans* et les mélanges de ceux-ci.

2. Composé de formule (I) selon la revendication 1, tel qu'il répond à la formule (IA) : 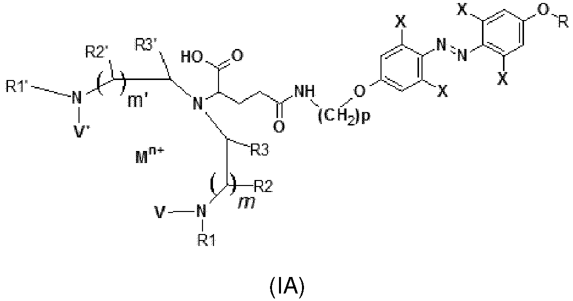 Dans laquelle :
M, n, R1, R2, R3, V, m, R1', R2', R3', V', m', p sont définis comme en formule (I) et
X est choisi parmi les atomes d'hydrogène et d'halogène ;
R est un groupe C1-C12 alkyle linéaire ou ramifié ;
La double liaison N=N est sous forme *cis* ou *trans ;*
Etant entendu que pour assurer la neutralité de la molécule, les n charges cationiques de M peuvent être neutralisées par de 0 à n groupes carboxylate (-COOH) substitués sur R1, R1', R2, R2', R3, R3', V, V', et/ou de 0 à n des contre-ions présents en solution le cas échéant.

3. Composé selon la revendication 1 ou 2 répondant à la formule générale (IB) : 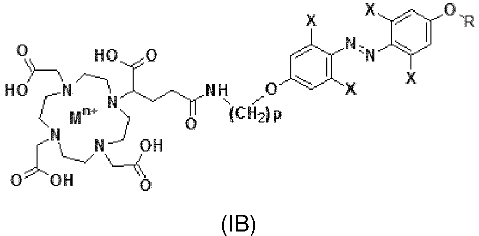 dans laquelle
M, n, p, X et R sont définis comme dans la revendication 1 ou 2.

4. Composé selon l'une quelconque des revendications précédentes tel qu'il répond à la formule générale (IC) : 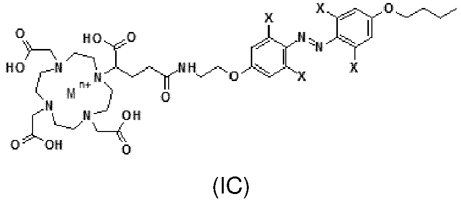 dans laquelle
M, n, X sont définis comme dans les revendications précédentes.

5. Procédé de préparation d'un composé selon l'une quelconque des revendications précédentes comprenant :
- l'étape de complexation du composé de formule (II) : 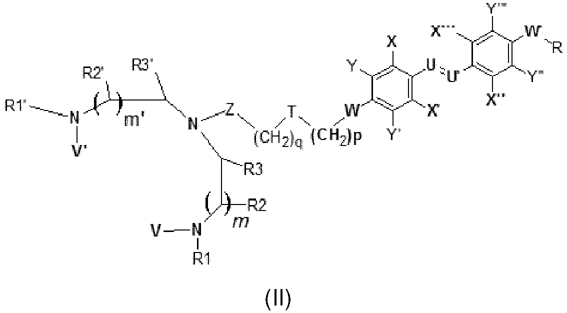 Dans laquelle :
R1, R2, R3, V, m, R1', R2', R3', V', m', Z, T, q, p, W, W', X, X', X", X‴, Y, Y', Y", Y‴, R, U, U' sont tels que définis en revendication 1 à 4,
Sous forme d'isomère *cis* et/ou *trans* et les mélanges de ceux-ci ;
avec un précurseur du métal M, et
- l'irradiation UV éventuelle du produit obtenu afin d'obtenir l'isomère *cis* majoritairement.

6. Procédé selon la revendication 5 tel que le composé de formule (II) est obtenu par couplage d'un composé de formule (III) et d'un composé de formule (IV) : 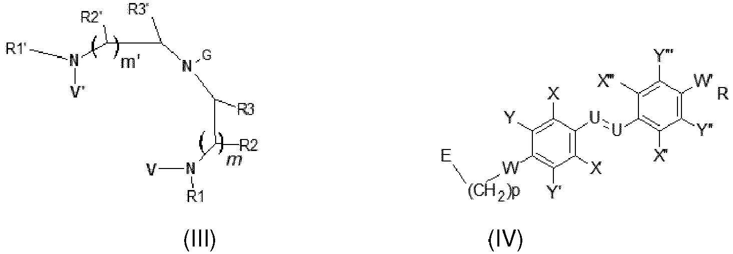 Dans lesquelles :
R1, R2, R3, V, m, R1', R2', R3', V', m', Z, T, q, p, W, W', X, X', X", X‴, Y, Y', Y", Y‴, R, U, U' sont tels que définis en revendication 1 à 4 ; et
E est une chaîne alkoxy ou alkyle en C1-C10 linéaire ou ramifiée, un groupe cycloalkyle ou aryle, comprenant éventuellement un ou plusieurs hétéroatomes choisis parmi N, O ou S, éventuellement intégrant ou substituée par un ou plusieurs substituants choisis indépendamment parmi les atomes d'halogène, et les groupes anhydride, carboxy, nitrile, nitro, thio, amino, amido, aryle, hétéroaryle, hydroxyle, ester, acide carboxylique ou carboxylate ; étant entendu que E contient un substituant nucléophile tel qu'une fonction hydroxyle, thiol ou une amine primaire ou secondaire pour effectuer le couplage sur la molécule (III) ;
G est une chaîne alkoxy ou alkyle en C1-C10 linéaire ou ramifiée, un goupe cylcoalkyle ou aryle, comprenant éventuellement un ou plusieurs hétéroatomes choisis parmi N, O ou S, éventuellement substituées par un ou plusieurs substituants choisis indépendamment parmi les atomes d'halogène, et les groupes anhydride, carboxy, nitrile, nitro, thio, amino (primaire ou secondaire), amido, aryle, hétéroaryle, hydroxyle, acide carboxylique ou carboxylate, étant entendu que G contient un substituant électrophile tel qu'une fonction acide carboxylique activée ou une fonction anhydride pour effectuer le couplage sur la molécule (IV).

7. Composition pharmaceutique comprenant un composé de formule (I) tel que défini selon l'une quelconque des revendications 1 à 4 majoritairement sous forme *cis,* et au moins un excipient pharmaceutiquement acceptable.

8. Composé de formule (I) tel que défini selon l'une quelconque des revendications 1 à 4 pour son utilisation pour le traitement du cancer tels que le cancer du poumon, le cancer du pancréas, le cancer du foie, le cancer de la rate, le carcinome pulmonaire à petites cellules, le cancer de la prostate, le rhabdomyosarcome, le cancer de l'estomac, le cancer gastrointestinal, le cancer colorectal, le cancer du rein, le cancer du sein, le cancer des ovaires, le cancer du testicule, le cancer de la thyroïde, le cancer de la tête et du cou, le cancer de la peau, le sarcome des tissus mous, le carcinome de la vessie, les cancers osseux, le myélome, le plasmocytome, le cancer des cellules germinales, le cancer de l'utérus, les leucémies, les lymphomes, le neuroblastome, le ostéosarcome, le rétinoblastome, les cancers du système nerveux central, les tumeurs de Wilms.

9. Composé de formule (I) pour son utilisation selon la revendication 8 comprenant l'administration du composé (I) majoritairement sous forme *cis* et l'irradiation de la tumeur par un rayonnement ionisant, tel que notamment choisi parmi les rayons X, les rayons gamma, les faisceaux d'électrons et d'hadrons, tels que les protons ou ions carbone.

10. Composé de formule (I) pour utilisation selon l'une quelconque des revendications 8 à 9 comprenant en outre le suivi dudit traitement par imageries médicales *in vivo* telles que l'IRM, la TEP, le scanner X ou la TEMP.

11. Composé de formule (I) pour utilisation selon l'une quelconque des revendications 8 à 10 comprenant en outre l'administration d'un ou plusieurs agents anti-cancéreux, tels qu'une chimiothérapie ou une immunothérapie comme les inhibiteurs de checkpoints immunitaires tels que les anti-CTLA4, anti-PD-L1 et anti-PD1.

## Patentansprüche

1. Eine Verbindung der Formel (I): 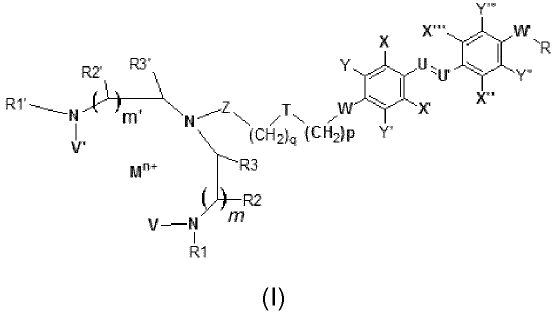 In dem:
M ein Metallatom ausgewählt aus Ce(III), Pr(III), Nd(III), Sm(III), Eu(III), Gd(III), Tb(III), Dy(III), Ho(III), Er(III), Tm(III), Yb(III), Mg(II), Ca(II), Mn(II), Fe(II), Fe(III), Cu(II), Zn(II), Ga(III), Y(III), Zr(III), Tc(IV), Tc (VI), Tc (VII), Ru(II), Ru(III), Ru(IV), Pd(II), Ag(I), In(III), Hf (IV), Re(VI), W(II), W(III), W(IV), W(V), W(VI), Os(III), Os(IV), Ir(III), Ir (IV), Pt(II), Au(I), Au (III), TI(III), Zr(IV), Nb(III), Bi(III);
n ist 1, 2, 3, 4, 5, 6 oder 7;
V und V', die gleich oder verschieden sein können, sind Wasserstoffatome oder lineare oder verzweigte C1-C10-Alkyl- oder Alkoxyketten oder C1-C10-Alkylketten, die miteinander verbunden sind und ein oder mehrere Heteroatome, ausgewählt aus N, O oder S, umfassen, die gegebenenfalls mit einem oder mehreren Substituenten, unabhängig voneinander ausgewählt aus Halogenatomen und Nitril-, Nitro-, Thio-, Amino-, Amido-, Aryl-, Heteroaryl-, Hydroxyl-, Carbonsäure- oder Carboxylatgruppen, substituiert sind, um einen Ring zu bilden;
R1, R1', R2, R2', die gleich oder verschieden sein können, sind Wasserstoffatome oder lineare oder verzweigte C1-C10-Alkyl- oder Alkoxyketten, die gegebenenfalls mit einem oder mehreren Substituenten, unabhängig voneinander ausgewählt aus Halogenatomen und Nitril-, Nitro-, Thio-, Amino-, Amido-, Aryl-, Heteroaryl-, Hydroxyl-, Carbonsäure- oder Carboxylatgruppen, substituiert sind;
R3 und R3', die gleich oder verschieden sein können, sind Wasserstoffatome oder lineare oder verzweigte C1-C10-Alkyl- oder Alkoxyketten, die gegebenenfalls mit einem oder mehreren Substituenten, unabhängig voneinander ausgewählt aus Halogenatomen und Nitril-, Nitro-, Thio-, Amino-, Amido-, Aryl-, Heteroaryl-, Hydroxyl-, Carbonsäure- oder Carboxylatgruppen, substituiert sind, oder R3 und R3' sind miteinander verbunden, um einen 5- bis 14-gliedrigen Heterocyclus oder Heteroaryl zu bilden;
m und m', die gleich oder verschieden sein können, sind gleich 1 oder 2;
X, X', X", X‴, Y, Y', Y", Y‴, die gleich oder verschieden sein können, unabhängig voneinander ausgewählt sind aus H; Halogenatomen; Alkoxy-, Alkyl- oder Cycloalkylgruppen, die gegebenenfalls unterbrochen oder substituiert sind mit einem oder mehreren Heteroatom(en) oder einer oder mehreren Gruppen COOH, CONH₂ , COSH, OH, NH₂ , SH; 5- bis 12-gliedrige Aryl- oder Heteroarylgruppen, gegebenenfalls substituiert mit einer oder mehreren COOH-, CONH₂ , COSH-Gruppen; COOH- oder NH₂ -Gruppen;
Z eine Alkylgruppe, die gegebenenfalls unterbrochen oder substituiert ist mit einem oder mehreren Heteroatom(en) oder einer COOH-, CONH₂ , COSH-, OH-, NH₂ , SH-Gruppe; eine 5- bis 12-gliedrige Aryl- oder Heteroarylgruppe, die gegebenenfalls substituiert ist mit einer oder mehreren COOH-, CONH₂ , COSH-Gruppen; oder eine COOH- oder NH₂ -Gruppe darstellt;
W und W', die gleich oder verschieden sein können, stellen unabhängig voneinander eine Gruppe CH₂; oder eine Aryl- oder Cycloalkylgruppe; ein Sauerstoff- oder Stickstoffatom (sekundär oder ternär); eine Amidbindung; eine Esterbindung; eine Thioetherbindung dar;
U und U', die gleich oder verschieden sein können, stellen die Gruppe CH oder NH dar, wobei die Doppelbindung U=U' in cis- oder trans-Form vorliegt;
T eine CH₂ -Gruppe; eine -C(=O)NH-Gruppe; eine Alkoxy-, Alkyl- oder Cycloalkylgruppe, die gegebenenfalls unterbrochen oder substituiert ist mit einem oder mehreren Heteroatom(en) oder einer COOH-, CONH₂ -, COSH-, OH-, NH₂ -, SH-Gruppe; eine 5- bis 12-gliedrige Aryl- oder Heteroarylgruppe, die ein oder mehrere Heteroatom(e) enthält und/oder gegebenenfalls substituiert ist mit einer oder mehreren Gruppen, ausgewählt aus COO-Alkyl, CONH-Alkyl, COSHAlkyl;
R steht für H oder eine lineare oder verzweigte C1-C18-Alkyl- oder Alkoxykette, die gegebenenfalls mit einem oder mehreren Substituenten substituiert ist, die unabhängig voneinander aus Halogenatomen und Nitril-, Nitro-, Thio-, Amino-, Amido-, Aryl-, Heteroaryl-, Hydroxyl-, Carbonsäure- oder Carboxylatgruppen ausgewählt sind;
p und q sind ganze Zahlen zwischen 0 und 6, insbesondere zwischen 1 und 6;
Um die Neutralität des Moleküls zu gewährleisten, können die n kationischen Ladungen von M durch 0 bis n Carboxylatgruppen (-COOH) neutralisiert werden, die gegebenenfalls an R1, R1', R2, R2', R3, R3', V, V' substituiert sind, und/oder durch 0 bis n der in Lösung befindlichen Gegenionen, falls erforderlich;
In Form von cis- und/oder trans-Isomeren und deren Mischungen.

2. Eine Verbindung der Formel (I) nach Anspruch 1, die die Formel (IA) hat: 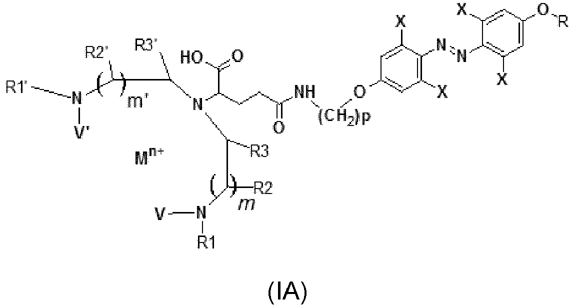 In dem:
M, n, R1, R2, R3, V, m, R1', R2', R3', V', m', p wie in Formel (I) definiert sind und X ist ausgewählt aus Wasserstoff- und Halogenatomen;
R ist eine lineare oder verzweigte C1-C12-Alkylgruppe;
Die N=N-Doppelbindung liegt in cis- oder trans-Form vor;
Um die Neutralität des Moleküls zu gewährleisten, können die n kationischen Ladungen von M durch 0 bis n Carboxylatgruppen (-COOH), die an R1, R1', R2, R2', R3, R3', V, V' substituiert sind, und/oder durch 0 bis n der in Lösung befindlichen Gegenionen neutralisiert werden, falls erforderlich.

3. Eine Verbindung nach Anspruch 1 oder 2 mit der allgemeinen Formel (IB): 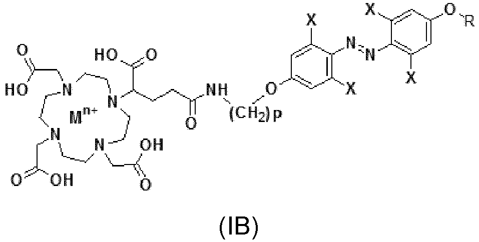 in dem
M, n, p, X und R sind wie in Anspruch 1 oder 2 definiert.

4. Verbindung nach einem der vorangehenden Ansprüche mit der allgemeinen Formel (IC): 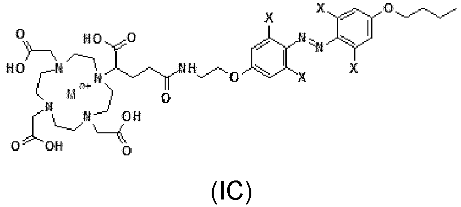 in dem
M, n, X sind wie in den vorhergehenden Ansprüchen definiert.

5. Verfahren zur Herstellung einer Verbindung nach einem der vorangehenden Ansprüche, umfassend:
- die Stufe der Komplexierung der Verbindung der Formel (II): 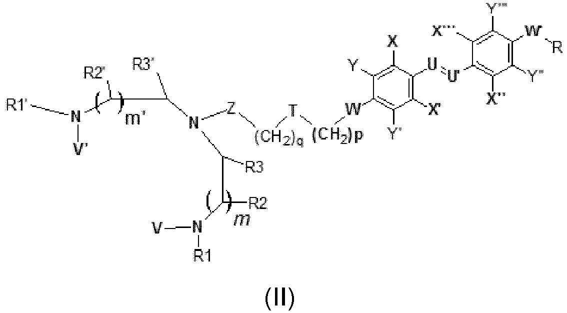 In dem:
R1, R2, R3, V, m, R1', R2', R3', V', m', Z, T, q, p, W, W', X, X', X", X'", Y, Y', Y", Y‴, R, U, U' sind wie in Anspruch 1 bis 4 definiert.
In Form von cis- und/oder trans-Isomeren und deren Mischungen;
mit einem Vorläufer des Metalls M, und
- die mögliche UV-Bestrahlung des erhaltenen Produkts, um überwiegend das cis-Isomer zu erhalten.

6. Verfahren nach Anspruch 5, bei dem die Verbindung der Formel (II) durch Kupplung einer Verbindung der Formel (III) und einer Verbindung der Formel (IV) erhalten wird: 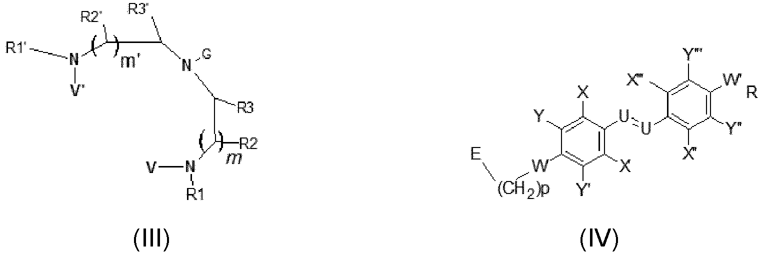 In dem:
R1, R2, R3, V, m, R1', R2', R3', V', m', Z, T, q, p, W, W', X, X, X', X", X'", Y, Y', Y", Y‴, R, U, U' sind wie in Anspruch 1 bis 4 definiert; und
E ist eine geradkettige oder verzweigte C1-C10-Alkyl- oder -Alkoxykette, eine Cycloalkyl- oder Arylgruppe, die gegebenenfalls ein oder mehrere Heteroatome, ausgewählt aus N, O oder S, enthält, gegebenenfalls integriert oder substituiert mit einem oder mehreren Substituenten, unabhängig ausgewählt aus Halogenatomen und Anhydrid, Carboxy-, Nitril-, Nitro-, Thio-, Amino-, Amido-, Aryl-, Heteroaryl-, Hydroxyl-, Ester-, Carbonsäure- oder Carboxylatgruppen, mit der Maßgabe, daß E einen nukleophilen Substituenten wie eine Hydroxyl-, Thiol- oder primäre oder sekundäre Aminfunktion enthält, um die Kopplung an das Molekül (III) zu bewirken;
G eine geradkettige oder verzweigte C1-C10-Alkyl- oder -Alkoxykette, eine Cycloalkyl- oder Arylgruppe ist, die gegebenenfalls ein oder mehrere Heteroatome, ausgewählt aus N, O oder S, enthält, die gegebenenfalls mit einem oder mehreren Substituenten, unabhängig voneinander ausgewählt aus Halogenatomen, und Anhydrid-, Carboxy-, Nitril-, Nitro-, Thio-, Carboxy-, Nitril-, Nitro-, Thio-, Amino- (primär oder sekundär), Amido-, Aryl-, Heteroaryl-, Hydroxyl-, Carbonsäure- oder Carboxylatgruppen substituiert sind, wobei es sich versteht, dass G einen elektrophilen Substituenten wie eine aktivierte Carbonsäurefunktion oder eine Anhydridfunktion enthält, um die Kopplung an das Molekül (IV) zu bewirken.

7. Pharmazeutische Zusammensetzung, umfassend eine Verbindung der Formel (I), wie in einem der Ansprüche 1 bis 4 definiert, vorwiegend in cis-Form, und mindestens einen pharmazeutisch annehmbaren Hilfsstoff.

8. Verbindung der Formel (I), wie in einem der Ansprüche 1 bis 4 definiert, zur Verwendung bei der Behandlung von Krebs, wie Lungenkrebs, Bauchspeicheldrüsenkrebs, Leberkrebs, Milzkrebs, kleinzelligem Lungenkarzinom, Prostatakrebs, Rhabdomyosarkom, Magenkrebs, Magen-Darm-Krebs, kolorektalem Krebs, Nierenkrebs, Brustkrebs, Eierstockkrebs, Hodenkrebs, Schilddrüsenkrebs, Kopf- und Halskrebs, Hautkrebs, Weichteilsarkom, Blasenkrebs, Knochenkrebs, Myelom, Plasmozytom, Keimzellkrebs, Gebärmutterkrebs, Leukämie, Lymphom, Neuroblastom, Osteosarkom, Retinoblastom, Krebserkrankungen des zentralen Nervensystems, Wilms-Tumore.

9. Verbindung der Formel (I) zur Verwendung nach Anspruch 8, umfassend die Verabreichung der Verbindung (I) hauptsächlich in cis-Form und die Bestrahlung des Tumors mit ionisierender Strahlung, wie sie insbesondere aus Röntgenstrahlen, Gammastrahlen, Elektronen- und Hadronenstrahlen, wie Protonen oder Kohlenstoffionen, ausgewählt wird.

10. Verbindung der Formel (I) zur Verwendung nach einem der Ansprüche 8 bis 9, ferner umfassend die Überwachung der Behandlung durch medizinische In-vivo-Bildgebung wie MRI, PET, Röntgen oder SPECT.

11. Verbindung der Formel (I) zur Verwendung nach einem der Ansprüche 8 bis 10, die ferner die Verabreichung eines oder mehrerer Anti-Krebs-Mittel, wie Chemotherapie oder Immuntherapie, wie Immun-Checkpoint-Inhibitoren, wie Anti-CTLA4, Anti-PD-L1 und Anti-PD1, umfasst.

## Claims

1. A compound of formula (I): 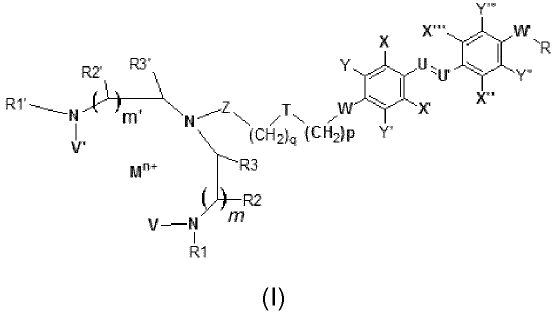 In which:
M represents a metal atom selected from Ce(III), Pr(III), Nd(III), Sm(III), Eu(III), Gd(III), Tb(III), Dy(III), Ho(III), Er(III), Tm(III), Yb(III), Mg(II), Ca(II), Mn(II), Fe(II), Fe(III), Cu(II), Zn(II), Ga(III), Y(III), Zr(III), Tc(IV), Tc (VI), Tc (VII), Ru(II), Ru(III), Ru(IV), Pd(II), Ag(I), In(III), Hf (IV), Re(VI), W(II), W(III), W(IV), W(V), W(VI), Os(III), Os(IV), Ir(III), Ir (IV), Pt(II), Au(I), Au (III), TI(III), Zr(IV), Nb(III), Bi(III); n is 1, 2, 3, 4, 5, 6, or 7;
V and V', which may be identical or different, are hydrogen atoms or linear or branched C1-C10 alkyl or alkoxy chains, or C1-C10 alkyl chains linked together and comprising one or more heteroatoms selected from N, O, or S, optionally substituted with one or more substituents independently selected from halogen atoms, and nitrile, nitro, thio, amino, amido, aryl, heteroaryl, hydroxyl, carboxylic acid or carboxylate groups, to form a ring;
R1, R1', R2, R2', which may be identical or different, are hydrogen atoms, or linear or branched C1-C10 alkyl or alkoxy chains optionally substituted with one or more substituents independently selected from halogen atoms, and nitrile, nitro, thio, amino, amido, aryl, heteroaryl, hydroxyl, carboxylic acid or carboxylate groups;
R3 and R3', which may be identical or different, are hydrogen atoms or linear or branched C1-C10 alkyl or alkoxy chains optionally substituted with one or more substituents independently selected from halogen atoms, and nitrile, nitro, thio, amino, amido, aryl, heteroaryl, hydroxyl, carboxylic acid or carboxylate groups, or R3 and R3' are linked together to form a 5- to 14-membered heterocycle or heteroaryl;
m and m', which may be identical or different, are equal to 1 or 2;
X, X', X", X‴, Y, Y', Y", Y‴, which may be identical or different, are independently selected from H; halogen atoms; alkoxy, alkyl or cycloalkyl groups optionally interrupted or substituted with one or more heteroatom(s) or group(s) COOH, CONH₂, COSH, OH, NH₂, SH; 5- to 12-membered aryl or heteroaryl groups optionally substituted with one or more COOH, CONH₂, COSH groups; COOH or NH₂ groups;
Z represents an alkyl group optionally interrupted or substituted with one or more heteroatom(s) or a COOH, CONH₂, COSH, OH, NH₂, SH group; a 5- to 12-membered aryl or heteroaryl group optionally substituted with one or more COOH, CONH₂, COSH groups; or a COOH or NH₂ group;
W and W, which may be the same or different, independently represent a CH₂ group; or an aryl or cycloalkyl group; an oxygen or nitrogen atom (secondary or ternary); an amide linkage; an ester linkage; a thioether linkage;
U and U', which may be the same or different, represent the CH or NH group, it being understood that the double bond U=U' is in *cis* or *trans* form;
T represents a CH₂ group; a -C(=O)NH group; an alkoxy, alkyl or cycloalkyl group optionally interrupted or substituted with one or more heteroatom(s) or a COOH, CONH₂, COSH, OH, NH₂, SH group; a 5- to 12-membered aryl or heteroaryl group containing one or more heteroatom(s) and/or optionally substituted with one or more groups chosen from COOalkyl, CONHalkyl, COSHalkyl;
R represents H, or linear or branched C1-C18 alkyl or alkoxy chain, optionally substituted with one or more substituents independently selected from halogen atoms, and nitrile, nitro, thio, amino, amido, aryl, heteroaryl, hydroxyl, carboxylic acid or carboxylate groups;
p and q are integers between 0 and 6, in particular between 1 and 6;
It being understood that in order to ensure the neutrality of the molecule, the n cationic charges of M can be neutralized by 0 to n carboxylate groups (-COOH) optionally substituted on R1, R1', R2, R2', R3, R3', V, V', and/or by 0 to n of the counterions present in solution if necessary;
In the form of *cis* and/or *trans* isomers and mixtures thereof.

2. A compound of formula (I) according to claim 1, as having the formula (IA): 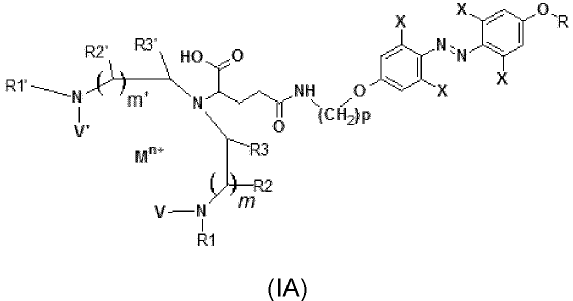 In which:
M, n, R1, R2, R3, V, m, R1', R2', R3', V', m', p are defined as in formula (I) and X is selected from hydrogen and halogen atoms;
R is a linear or branched C1-C12 alkyl group;
The N=N double bond is in *cis* or *trans* form;
It being understood that in order to ensure the neutrality of the molecule, the n cationic charges of M can be neutralized by 0 to n carboxylate groups (-COOH) substituted on R1, R1', R2, R2', R3, R3', V, V', and/or by 0 to n of the counterions present in solution if necessary.

3. A compound according to claim 1 or 2 having the general formula (IB): 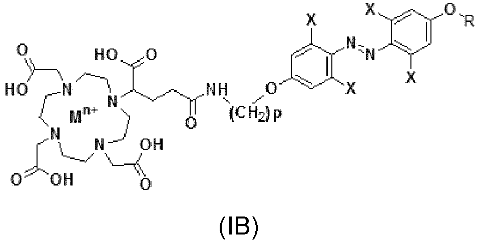 in which
M, n, p, X and R are defined as in claim 1 or 2.

4. A compound according to any of the preceding claims as having the general formula (IC): 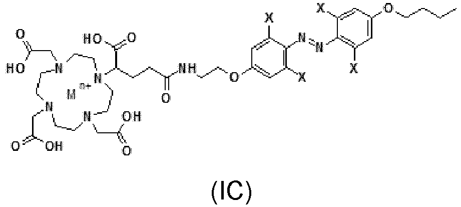 in which
M, n, X are defined as in the preceding claims.

5. A method for preparing a compound according to any of the preceding claims comprising:
- the step of complexing the compound of formula (II): 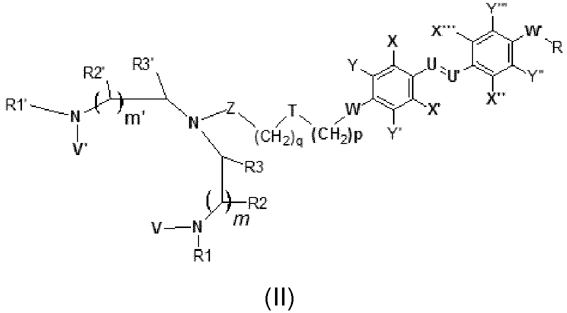 In which:
R1, R2, R3, V, m, R1', R2', R3', V', m', Z, T, q, p, W, W, X, X', X", X'", Y, Y', Y", Y‴, R, U, U' are as defined in claim 1 to 4.
In the form of *cis* and/or *trans* isomers and mixtures thereof;
with a precursor of the metal M, and
- the possible UV irradiation of the product obtained, in order to predominantly obtain the *cis* isomer.

6. A method according to claim 5 such that the compound of formula (II) is obtained by coupling a compound of formula (III) and a compound of formula (IV): 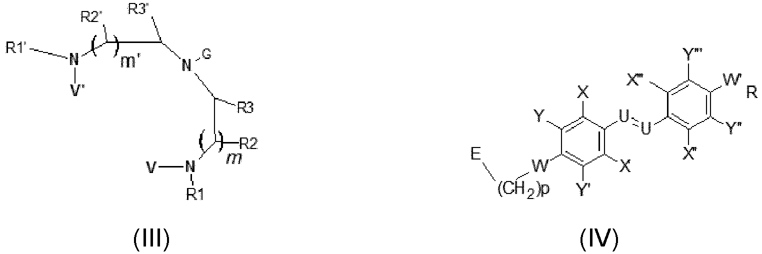 In which:
R1, R2, R3, V, m, R1', R2', R3', V', m', Z, T, q, p, W, W, X, X', X", X'", Y, Y', Y", Y‴, R, U, U' are as defined in claim 1 to 4; and
E is a straight or branched C1-C10 alkyl or alkoxy chain, a cycloalkyl or aryl group, optionally comprising one or more heteroatoms selected from N, O or S, optionally integrating or substituted with one or more substituents independently selected from halogen atoms, and anhydride, carboxy, nitrile, nitro, thio, amino, amido, aryl, heteroaryl, hydroxyl, ester, carboxylic acid or carboxylate groups provided that E contains a nucleophilic substituent such as a hydroxyl, thiol or primary or secondary amine function to effect the coupling to the molecule (III);
G is a straight or branched C1-C10 alkyl or alkoxy chain, a cycloalkyl or aryl group, optionally comprising one or more heteroatoms selected from N, O or S, optionally substituted with one or more substituents independently selected from halogen atoms, and anhydride, carboxy, nitrile, nitro, thio, amino (primary or secondary), amido, aryl, heteroaryl, hydroxyl, carboxylic acid or carboxylate groups, it being understood that G contains an electrophilic substituent such as an activated carboxylic acid function or an anhydride function in order to carry out the coupling to the molecule (IV).

7. A pharmaceutical composition comprising a compound of formula (I) as defined in any one of claims 1 to 4 predominantly in cis form, and at least one pharmaceutically acceptable excipient.

8. A compound of formula (I) as defined in any of claims 1 to 4 for use in the treatment of cancer such as lung cancer, pancreatic cancer, liver cancer, spleen cancer, small cell lung carcinoma, prostate cancer, rhabdomyosarcoma, stomach cancer, gastrointestinal cancer, colorectal cancer, kidney cancer, breast cancer, ovarian cancer, testicular cancer, thyroid cancer, head and neck cancer, skin cancer, soft tissue sarcoma, bladder carcinoma, bone cancers, myeloma, plasmacytoma, germ cell cancer, uterine cancer, leukemia, lymphoma, neuroblastoma, osteosarcoma, retinoblastoma, central nervous system cancers, Wilms' tumors.

9. A compound of formula (I) for use according to claim 8, comprising administration of compound (I) mainly in cis form and irradiation of the tumor with ionizing radiation, such as those chosen in particular from X-rays, gamma rays, electron and hadron beams, such as protons or carbon ions.

10. A compound of formula (I) for use according to any of claims 8 to 9 further comprising monitoring said treatment by *in vivo* medical imaging such as MRI, PET, X-ray or SPECT.

11. A compound of formula (I) for use according to any of claims 8 to 10 further comprising administering one or more anti-cancer agents, such as chemotherapy or immunotherapy such as immune checkpoint inhibitors such as anti-CTLA4, anti-PD-L1 and anti-PD1.
